# EUROPEAN PATENT SPECIFICATION

(11) **EP 3 180 620 B1**
(45) Date of publication and mention of the grant of the patent: **01.01.2020**
(21) Application number: 15750708.8
(22) Date of filing: 12.08.2015
(51) Int. Cl.: G01N 33/569

(54) **PREDICTING S. AUREUS DISEASE**
VORHERSAGE DER S. AUREUS-KRANKHEIT
PRÉDICTION DE MALADIE DE S. AUREUS

(30) Priority: 12.08.2014 EP 14180606
(43) Date of publication of application: 21.06.2017
(73) Proprietor: X4 Pharmaceuticals (Austria) GmbH, 1030 Vienna (AT)
(72) Inventor: NAGY, Eszter, A-1070 Vienna (AT); STULIK, Lukas, A-1160 Vienna (AT); ROUHA, Harald, A-1220 Wien (AT); MAGYARICS, Zoltán, A-1110 Vienna (AT)
(74) Representative: Redl, Gerda
(86) International application number: PCT/EP2015/068536
(87) International publication number: WO 2016/023943

(56) References cited:
- WO-A2-2012/109285
- STULIK LUKAS ET AL: "[alpha]-Hemolysin activity of methicillin-susceptible Staphylococcus aureus predicts ventilator-associated pneumonia", AMERICAN JOURNAL OF RESPIRATORY AND CRITICAL CARE MEDICINE, AMERICAN THORACIC SOCIETY, UNITED STATES, vol. 190, no. 10, 15 November 2014 (2014-11-15), pages 1139-1148, XP008174843, ISSN: 1535-4970, DOI: 10.1164/RCCM.201406-1012OC
- ARROLIGA ALEJANDRO C ET AL: "Back to the future: [alpha]-hemolysin activity on blood agar to predict ventilator-associated pneumonia caused by Staphylococcus aureus", AMERICAN JOURNAL OF RESPIRATORY AND CRITICAL CARE MEDICINE, AMERICAN THORACIC SOCIETY, UNITED STATES, vol. 190, no. 10, 15 November 2014 (2014-11-15), pages 1086-1088, XP008174842, ISSN: 1535-4970, DOI: 10.1164/RCCM.201410-1886ED
- ANA TAVARES ET AL: "Insights into Alpha-Hemolysin (Hla) Evolution and Expression among Staphylococcus aureus Clones with Hospital and Community Origin", PLOS ONE, vol. 9, no. 7, 17 July 2014 (2014-07-17), page e98634, XP055169077, DOI: 10.1371/journal.pone.0098634
- DENNIS L STEVENS ET AL: "Impact of Antibiotics on Expression of Virulence- Associated Exotoxin Genes in Methicillin-Sensitive and Methicillin-Resistant Staphylococcus aureus", THE JOURNAL OF INFECTIOUS DISEASES, vol. 11, 18 December 2006 (2006-12-18), pages 195-202, XP055168873,
- A. CONWAY MORRIS ET AL: "Diagnostic importance of pulmonary interleukin-1 and interleukin-8 in ventilator-associated pneumonia", THORAX, vol. 65, no. 3, 12 October 2009 (2009-10-12), pages 201-207, XP055168881, ISSN: 0040-6376, DOI: 10.1136/thx.2009.122291
- NELE BRUSSELAERS ET AL: "Value of lower respiratory tract surveillance cultures to predict bacterial pathogens in ventilator-associated pneumonia: systematic review and diagnostic test accuracy meta-analysis", INTENSIVE CARE MEDICINE, vol. 39, no. 3, 1 March 2013 (2013-03-01), pages 365-375, XP055169057, ISSN: 0342-4642, DOI: 10.1007/s00134-012-2759-x
- CHEUNG G Y C AND OTTO M: "The potential use of toxin antibodies as a strategy for controlling acute Staphylococcus aureus infections", EXPERT OPINION ON THERAPEUTIC TARGETS, INFORMA HEALTHCARE, UK, vol. 16, no. 6, 1 June 2012 (2012-06-01), pages 601-612, XP009175171, ISSN: 1472-8222, DOI: 10.1517/14728222.2012.682573

## Description

The present invention relates to a method for the prediction of ventilator-associated pneumonia (VAP) in a ventilated subject heavily colonized by methicillin susceptible Staphylococcus aureus (MSSA) and asymptomatic, as defined in the appended claims.

### BACKGROUND

*Staphylococcus aureus* is normally found as a commensal species on the skin or in the nose of people and several animal species. Implied by their commensal characteristics, asymptomatic carriage of *S. aureus* is generally considered as harmless, whereas typical infections can range from relatively minor skin diseases to life-threatening invasive infections.

Historically, infections are treated by broad-spectrum antibiotics, such as methicillin. Due to a broad usage of antibiotics, certain multi-drug resistant strains have emerged that are resistant to standard of care therapy such as treatment with methicillin and other beta-lactam antibiotics e.g. penicillin and cephalosporins. They are referred to as methicillin-resistant *Staphylococcus aureus* (also known as multi-drug resistant *Staphylococcus aureus,* or "MRSA") and differentiated from methicillin susceptible strains (MSSA).

*S. aureus* infections, including MRSA, are causative for potentially life-threatening diseases affecting a broad range of bodysites, including skin, soft tissue, bones, joints, surgical wounds, the bloodstream, heart valves, lungs, or other organs. Thus, *S. aureus* infections can result in disease conditions associated therewith, which are potentially fatal diseases, such as necrotizing fasciitis, endocarditis, sepsis, toxic shock syndrome, and various forms of pneumonia, including necrotizing pneumonia, and toxin production in furunculosis and carbunculosis. *S. aureus* infections are especially difficult to manage in hospital or tertiary-care home settings where patients are often immune-compromised, subjected to (trauma) surgery or other treatment implying the use of invasive devices such as catheters, drainages and tracheal-tubes. Such patients are at far greater risk for infection than the general public.

Mechanically ventilated patients are at high risk of bacterial colonization that may progress to ventilator-associated respiratory infections, manifested as tracheobronchitis (VAT) or pneumonia (VAP). Both conditions contribute to prolonged ventilation and stay in the intensive care unit (ICU), increased health-care costs and VAP is associated with increased mortality [1-4]. Recently, a new approach to disease management has been implemented using serial microbial analysis of endotracheal aspirates (ETAs), which is performed to identify and quantify bacteria colonizing the lower airways. Heavy colonization defined as >+++ (or 3+) growth on semi-quantitative endotracheal aspirates (SQ-ETA) or quantitative ETA ≥ 10⁵ colony forming units (CFU)/ml of a respiratory pathogen(s) is a risk factor for progression to VAT and/or VAP [4-6]. Detection of specific bacterial pathogens helps to guide earlier, targeted antibiotic treatment and antibiotic stewardship efforts [7].

*S. aureus* virulence and pathogenesis have been extensively studied in animal models, but the role of virulence factors in the pathogenesis of human disease is poorly understood [8,9]. Identification of relevant virulence factors and biomarkers would support more effective prevention strategies, and initiation of earlier therapy of high-risk patients.

One of the hallmarks of the *S. aureus* pathogenesis is the production of a multitude of secreted toxins (exotoxins, cytotoxins or cytolysins) that can kill several different cell types including erythrocytes, neutrophils, granulocytes and other immune cells, as well as epithelial cells of the lung or skin. Moreover, most of these toxins activate immune cells and act as potent pro-inflammatory signals. A prominent member of *S. aureus* cytotoxins is alpha haemolysin (Hla, also referred to as alpha-toxin, α-hemolysin α-toxin), which is highly potent in lysing human bronchial and lung alveolar epithelial cells, as well as macrophages and lymphocytes, and is implicated in the induction of pro-inflammatory processes [10]. Its dominant role in the pathogenesis of *S. aureus* pneumonia has been demonstrated in several different animal models [10]. Hla has also shown promise as a vaccine antigen and monoclonal antibody target in proof-of-concept animal models of several *S*. *aureus* diseases, and is currently being evaluated in human trials of both active and passive immunization [11-14].

WO2013/156534 A1 describes *S*. *aureus* antibodies which are cross-reactive binding to alpha-toxin and at least one of the bi-component toxins of *S. aureus.*

WO2012/109285 A2 describes antibodies specifically binding *S. aureus* alpha-toxin which may be used in a kit, e.g. an enzyme-linked immunosorbent assay (ELISA).

WO2011/018208 A1 describes human monoclonal antibodies against *S. aureus* alpha-toxin and its use in treating or preventing abscess formation.

WO2010/121298 A1 describes methods of detecting methicillin-sensitive (MSSA) and/or methicillin-resistant *Staphylococcus aureus* (MRSA), using a primer complementary to a sequence in *S*. *aureus* nucleic acid of the *orfx* gene, and another primer complementary to a sequence in *S*. *aureus* nucleic acid of the *orfx* gene and also complementary to a sequence in *S*. *aureus* nucleic acid of SCCmec cassette.

WO2010089569 A1 describes methods for the detection or diagnosis of a bacterial infection or colonisation utilising mass spectrometric analysis, in particular mass spectrometric analysis of biomarker profiles, such as the biomarker profile for *S. aureus.*

Airway colonization by *S. aureus* is often a precursor for the development of MRSA or MSSA induced ventilator-associated tracheobronchitis (VAT) and/or pneumonia (VAP). However, little is known about the pathogen-associated factors of *S. aureus* that promote progression from colonization to pneumonia. Detection of *S. aureus* isolates with a high propensity to cause VAP and identification of simple, sensitive and specific biomarkers would greatly support prophylaxis or initiation of earlier antibiotic therapy for VAT and VAP.

### SUMMARY OF THE INVENTION

The object of the present invention was to find a new biomarker to evaluate the risk of *S. aureus* disease occurence in a subject heavily colonized with *S. aureus,* before showing symptoms of disease. Specifically, it was the object to provide a test determining a marker indicating the risk to initiate appropriate therapy before onset of disease.

The object is achieved by the provision of the embodiments of the present invention.

According to the invention, there is provided a method for the prediction of ventilator-associated pneumonia (VAP) in a ventilated subject heavily colonized by methicillin susceptible *Staphylococcus aureus* (MSSA) and asymptomatic, comprising the step of determining the alpha haemolysin level in a biological sample of said subject as compared to a predetermined level, wherein an elevated alpha haemolysin level is indicative of the risk of VAP disease onset, wherein the predetermined alpha haemolysin level is higher than a reference value of a *S. aureus* strain expressing no alpha haemolysin.

Specifically, the subject is heavily colonized with *S. aureus* and asymptomatic or not showing symptoms of systemic disease. Such symptoms are specifically one or more of *S. aureus* infection or disease, in particular clinical symptoms leading to diagnosis of *S. aureus* disease, e.g. pneumonia, specifically ventilator-associated pneumonia (VAP), bronchitis, specifically ventilator-associated tracheobronchitis (VAT), sepsis, specifically severe sepsis, or chronic wound infection.

Exemplary clinical symptoms leading to the diagnosis of *S. aureus* disease in a subject heavily colonized with *S. aureus* are herein also referred to as *S. aureus* disease specific symptoms, and particularly for VAT and VAP, include leukocytosis, fever or hypothermia, purulent respiratory secretions, presence of a new or progressive infiltrate on a chest radiograph or CT scan. General symptoms of acute infection are defined by the APACHE-II score which covers all important aspects of acute infections (i.e. age, rectal temperature, mean arterial pressure, heart rate, arterial pH, respiratory rate, serum levels of sodium and potassium, creatinine, hematocrit, white blood cell count and Glasgow Coma Scale). The symptoms are considered *S. aureus* specific, if caused by *S. aureus,* e.g. by *S. aureus* colonization as determined by standard microbiological methods or culture.

The *S. aureus* disease specific symptoms are differentiated from other unspecific underlying physiological conditions that a *S. aureus* colonized patient may be suffering from. For example, a ventilated patient in the ICU may be suffering from conditions that result in a compromised immune system, for example malignancy or have undergone organ transplantation and may be considered at risk of developing *S. aureus* disease/infection if identified to be heavily colonized with *S.aureus.*

Specifically, the alpha haemolysin level is indicative of the likelihood of onset of *S. aureus* disease, which disease is specifically VAP. In particular, the alpha haemolysin level is indicative of the fast emergence of one or more symptoms leading to a diagnosis of such *S. aureus* disease.

The alpha haemolysin level is specifically the level of alpha haemolysin activity, such as determined in a functional or cell-based assay, or the alpha haemolysin gene expression or expression products, *e.g.* the level of translation or transcription products, such as mRNA, or those which are complementary to the nucleotide sequence of such expression products in the sense or complementary anti-sense, or alpha haemolysin protein or parts/fragments of the alpha haemolysin or reaction products of alpha haemolysin with any natural or artificial reagents. In particular, the level of alpha haemolysin is determined in a sample of the subject *ex vivo*, thus, by an appropriate *in vitro assay.*

The level is suitably determined as the content or concentration (w/v or w/w) or activity (unit/v or unit/w) of alpha haemolysin. As an alternative to numeric results of such determination, the alpha haemolysin level may as well be determined as a specific score or a specific level above a cut-off value or threshold or reference value, *e.g*., in semi-quantitative assays.

According to a specific aspect, the subject is heavily colonized by *S. aureus,* specifically defined by a level of ≥10⁵ CFU/mL in a biological sample of said subject and/or or moderate or heavy (+++ (3+) or ++++ (4+)) bacterial growth of *S. aureus* in a biological sample, such as an ETA fluid sample, as determined by standard microbiological tests.

Specifically, the subject is colonized by *S. aureus* and optionally at risk of suffering from *S. aureus* disease, as determined by a method of determining the presence of the bacterium or a bacterial marker indicative of of *S. aureus,* or the likely onset of *S. aureus* disease, preferably by a method of determining the gene expressing said marker, an expression product of said gene or using standard microbiological techniques. An exemplary method of standard microbiological techniques is the blood agar assay that demonstrates alpha haemolysin activity.

According to a specific aspect, the patient is colonized and/or infected with *S. aureus,* specifically MSSA.

Specifically, the subject is colonized in the nose or nasopharynx, and the subject is undergoing intubation. Based on the determination of the alpha haemolysin level as described herein, a highly hemolytic strain can be detected in the nose or nasopharynx prior to intubation, which allows the risk assessment of disease progression and specific pretreatment measures preventing colonization in the lower airways upon intubation. Though such colonization would normally be asymptomatically colonizing the nose or nasopharynx, the bacteria are potentially brought into the lower airways upon intubation rendering the subject at risk for developing heavy lower airway colonization and related disease. Likewise, the present method provides for the determination of the alpha haemolysin level as described herein and the detection of a highly hemolytic strain in the nose or nasopharynx of a subject that is already intubated allowing the risk assessment of disease progression.

Specifically, the subject is an intensive care unit (ICU) patient who is ventilated and optionally at risk of ventilator-associated infections such as VAT or VAP, or a patient at risk of skin and soft tissue infections (SSTI). Specifically, the subject is a mechanically ventilated patient and heavily colonized with *S. aureus* in the lower airways, such as in the trachea or the lung.

According to a specific aspect, the biological sample is a body fluid or tissue sample, preferably any sample selected from the group consisting of a blood sample, stool sample, skin sample, urine sample, cerebrospinal fluid, and a respiratory tract specimen such as endotracheal aspirates, pleural fluid, lung tap, nasal swab or sputum.

Specifically, the biological sample is treated to produce a *S. aureus* isolate originating from the biological sample, which isolate may be further characterized for its MSSA genotype or phenotype, and/or the level of alpha haemolysin expression. Preferable sample preparation methods for producing bacterial isolates are employing bacterial enrichment and cultivation steps.

Specifically, the biological sample is treated to determine the alpha haemolysin level directly in the sample, optionally following preparatory steps of enrichment or purification to reduce matrix effects and to increase the specificity and sensitivity of the test. Preparatory steps include culturing of the biological specimen according to standard culture procedures such as but not exclusively being hemocultures in standard bacterial growth media as well as the culturing of specimens on solid agar (including phenotyping - i.e. antibiogram) as performed in routine microbiology laboratories. Bacteria may be sub-cultured for expansion of CFU in different growth media (standard media and/or chemically defined media; high nutrient, low nutrient, limited growth media composition) to enhance expression of virulence factors (e.g. RPMI-CAS which is low in iron and resembles the *in vivo* conditions more faithfully than high nutrient media, therefore enhances the expression of exotoxins and other bacterial virulence factors). Bacterial suspensions may be prepared and washed in standard buffer solutions to remove potential matrix effects.

According to another specific aspect, said determination of the alpha haemolysin level comprises a semi-quantitative or quantitative measurement of alpha haemolysin expression or activity.

Specifically, the reference or control is a predetermined level of alpha haemolysin, or obtained by calibrating a reference or control value (e.g. a cut-off value or threshold or reference value) against a predetermined alpha haemolysin level obtained from a biological material, such as a comparable biological sample of a subject that has a predetermined risk of *S. aureus* disease, or obtained from a *S. aureus* strain that expresses a certain level of alpha haemolysin.

According to a specific aspect, said reference is a predetermined alpha haemolysin level indicative of disease or disease progression or obtained from a *S. aureus* strain expressing low, medium or high alpha haemolysin.

Specifically, said standard or reference control is a predetermined alpha haemolysin level indicative of the onset of *S. aureus* disease or disease progression, or obtained from a *S. aureus* strain expressing low, medium or high alpha haemolysin.

Specifically, the standard or reference control is a predetermined alpha haemolysin amount or activity which is higher than the alpha haemolysin level expressed by a *S. aureus* strain expressing no alpha haemolysin.

Preferably, specific strains are used to provide a reference, which are determined to be low expressors or have a hemolysis profile indicating a low level of hemolysis. Exemplary strains that express no or a low level of alpha haemolysin or no hemolytic activity which could serve as a reference or means to calibrate a test, are the following: No expression of alpha haemolysin: *S. aureus* strain designated MRSA252 (available from ATCC, catalogue reference ATCC BAA-1720). Low level of alpha haemolysin (1+): *S. aureus* strain designated Newman D2C (available from Pilic Health England Bacterial Collection, catalogue reference NCTC 10833).

It is preferred to use *S. aureus* strains as a reference to the method described herein or to calibrate the method described herein. Specifically, if the alpha haemolysin level in the biological sample is higher than the reference of a low expressor, such level is indicative of the onset of *S. aureus* disease. If the alpha haemolysin level in the biological sample is at least 2-fold higher than the reference of a low-expressor or a non-hemolytic strain, there is a strong indication of disease. If the determined level is at least 3-fold, or at least 4-fold, or at least 5-fold higher than the reference, the indication is even stronger, indicating a high risk of rapidly developing and progressing disease.

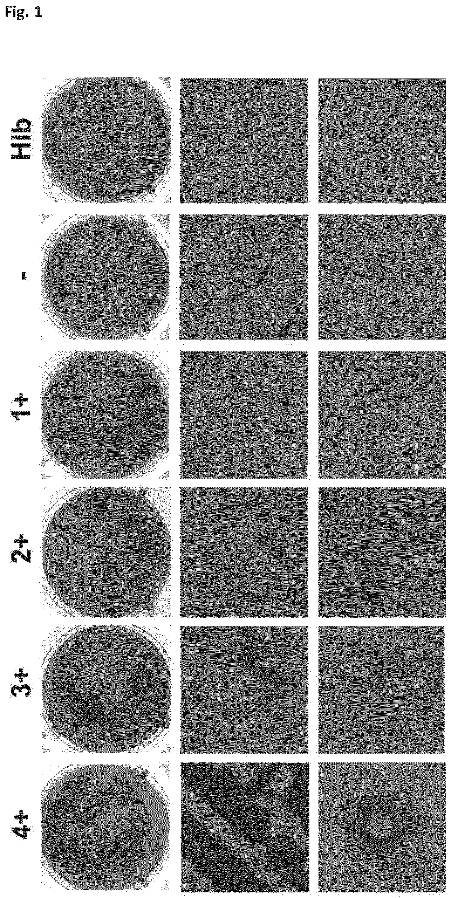

Based on the sheep-blood COS agar plating (Figure 1), a hemolysis profile of semi-quantitative evaluation of ≥2+ as compared to non-hemolytic isolates may be prepared, which could serve as an indicator for medium (2+) to high Hla (3+, 4+) expression and a strong marker of likely onset of *S. aureus* disease.

Reference material, such as a reference control may be conveniently included in an assay, such as by way of a reference standard, e.g. a standard alpha haemolysin preparation with a defined content or concentration of alpha haemolysin, which standard is used as internal or external control, or by way of a reference value, read-out or curve for comparison reason. The standard alpha haemolysin preparation may contain or essentially consist of purified alpha haemolysin (e.g. purified from a *S. aureus* strain or isolate) or a recombinant alpha haemolysin preparation.

Specifically, the level of alpha haemolysin is determined by at least one of an immunoassay, preferably any of ELISA, CIA, RIA, IRMA, agglutination assay, immunochromatography, specifically lateral flow immunochromatographic assays (such as dipstick tests), Western-blot, mass-spectrometry, nuclear magnetic resonance (NMR), or a method of determining corresponding DNA or RNA indicative of alpha haemolysin or the alpha haemolysin level, preferably employing a nucleic acid hybridization assay or a nucleic acid amplification assay.

According to a specific aspect, the method further comprises the measurement of at least one additional marker indicative of *S. aureus,* or *S. aureus* colonization or infection.

Specifically, said measurement of the at least one additional marker comprises the determination of a gene expressing any *S. aureus* specific bacterial component or the respective gene expression product of any *S. aureus* specific bacterial component, other than alpha haemolysin, preferably selected from the group consisting of
i) *S. aureus* Protein A;
ii) a *S. aureus* antibiotic resistance marker preferably at least any of PBP2a, the *mecA* gene or an expression product of the *mecA* gene;
iii) any other *S. aureus* antigen including a *S. aureus* cytotoxin, preferably Panton-Valentine leukocidin (PVL/LukSF), LukGH (LukAB), gamma-haemolysin, LukED; *S. aureus* virulence factors, secreted and surface proteins; or any *S. aureus* specific compounds such as lipoteichoic acid, and
iii) a combination of any of the bacterial components of i), ii) and/or iii), preferably determining as additional markers at least *S. aureus* Protein A and PBP2a.

Specifically, said measurement of the at least one additional marker comprises the determination of any of a gene expressing any cytotoxin, *S. aureus* virulence factors, secreted and surface proteins, or any *S. aureus* specific compounds, or the respective gene expression products, specifically *S. aureus* virulence factors, *S. aureus* antibiotic resistance markers and *S. aureus* specific bacterial components, and preferably at least any of PBP2a, the *mecA* gene or an expression product of the *mecA* gene or the *S. aureus* specific Protein A protein or gene encoding this protein.

The preferred assay combinations comprise tests for
a) disease biomarker, in particular alpha haemolysin; and
b) *S.aureus* detection (Protein A) and resistance marker (e.g. PBP2a); and optionally further markers.

Specifically, a quantitative or qualitative immunoassay or mass-spectrometry may be used to determine one or more markers, including the alpha haemolysin marker. When a further marker of *S. aureus,* the *S. aureus* disease or disease progression, or a marker of pathogen identification or resistance is determined besides the alpha haemolysin marker, a qualitative, semi-quantitative or quantitative assay may be used. Such determination may be consecutively, in parallel or simultaneously with the determination of the alpha haemolysin level, thus, in separate biological samples or in the same sample, reaction mixture or containment.

Specifically, a quantitative PCR assay or a semi-quantitative PCR assay may be used, optionally a multiplexed assay to determine the level of alpha haemolysin, in particular the level of genes or gene expression, and at least one further marker of disease or disease progression or characterization of the *S. aureus* in the sample (e.g. antibiotic resistance profile, confirmation of *S. aureus* presence).

Preferably further markers of *S. aureus* are determined which are associated with MSSA or MRSA. Such further markers are selected from the group consisting of penicillin-binding protein 2a (PBP2a), or its cognate gene (*mecA*) or an expression product of the *mecA* gene, which is a determinant for MRSA.

Preferably further markers of *S. aureus* are determined which are associated with *S. aureus* identification. Such further markers are selected from the group consisting cell-wall components such as lipoteichoic acid or wall-teichoic acid, or cell surface proteins, such as *S. aureus* specific Protein A.

Further described herein is the use of alpha haemolysin as an *in vitro* marker for the onset of ventilator-associated respiratory infections, tracheobronchitis or pneumonia, in a subject heavily colonized with *S. aureus* but not showing any symptom of *S. aureus* disease.

According to another specific aspect, the invention further provides for the *in vitro* use of alpha haemolysin as an *in vitro* marker for determining the risk of VAP disease in a ventilated asymptomatic subject heavily colonized by MSSA.

According to another specific aspect, the invention provides for the use of a diagnostic composition comprising a specific detection molecule for alpha haemolysin for determining the risk of VAP disease in a ventilated subject heavily colonized with MSSA, wherein the detection molecule is an alpha haemolysin binder selected from the group consisting of an antibody, antibody fragment, cellular receptor or ligand, or a nucleotide sequence hybridizing to the alpha haemolysin gene or an expression product of said gene.

Further described herein is the use of a specific detection molecule for alpha haemolysin for the preparation of a diagnostic composition for determining the disease onset of ventilator-associated respiratory infections, tracheobronchitis or pneumonia, in a subject heavily colonized with *S. aureus* but not showing any symptom of *S. aureus* disease.

Further described herein is the use of a diagnostic composition comprising a specific detection molecule for alpha haemolysin for determining the disease onset of ventilator-associated respiratory infections, tracheobronchitis or pneumonia, in a subject heavily colonized with *S. aureus* but not showing any symptoms of *S. aureus* disease.

Such diagnostic composition may be a reagent ready-to-use in a reaction mixture with the biological sample, or a conserved form of such reagent, e.g. a storage-stable form such as lyophilized; snap-frozen (e.g. in liquid nitrogen), ultra low-temperature storage (e.g. -70°C and -80°C), cold-storage (e.g. -20°C-5°C) and controlled room temperature (e.g. 15°C-27°C); standard sample storage as e.g. glycerol-stocks, tissue paraffin blocks, (buccal) swabs and other standard biological sample storage methods, which conserved form of a reagent can be reconstituted or prepared to obtain a ready-to-use reagent. Such reagent is typically in the form of an aqueous solution, specifically (physiological) buffer conditions (e.g. EDTA buffered, phosphate buffer, HBSS, citrate buffer etc.).

Specifically, the detection molecule is an alpha haemolysin binder selected from the group consisting of an antibody, antibody fragment, cellular receptor or ligand, or a nucleotide sequence hybridizing to the alpha haemolysin gene or an expression product of said gene. In this regard, hybridization conditions may be established according to conventional protocols.

Further described herein is the use of an alpha haemolysin diagnostic kit, in a method provided herein. For example, a kit for determining the alpha haemolysin level by standard microbiology techniques, e.g. including a blood agar plate may be used as described herein.

The alpha haemolysin diagnostic kit is preferably employing a specific detection molecule for alpha haemolysin, suitable for determining the alpha haemolysin level.

Further described herein is a new alpha haemolysin diagnostic kit, which is in the storage-stable form. Such kit preferably comprises all essential components to determine the alpha haemolysin level in the biological sample, optionally without common or unspecific substances or components, such as water, buffer or excipients. The storage stable kit can be stored preferably at least 6 months, more preferably at least 1 or 2 years. It may be composed of dry (e.g. lyophilized) components, and/or include preservatives.

Specifically, the alpha haemolysin diagnostic kit comprises one or more reagents capable of specifically reacting with the *S. aureus* alpha haemolysin expression product and/or a specific detection molecule suitable for determining the level of alpha haemolysin, which is provided in the storage-stable form, preferably as a packaged unit.

The preferred alpha haemolysin diagnostic kit is provided as a packaged or prepackaged unit, e.g. wherein the components are contained in only one package, which facilitates routine experiments. Such package may include the reagents necessary for one or more tests, e.g. suitable to perform the tests of a series of biological samples. The kit may further suitably contain a Hla preparation as a standard or reference control.

Specifically, provided herein is the use of a alpha haemolysin diagnostic kit in a method according to the invention, wherein the diagnostic kit comprises one or more reagents capable of specifically reacting with the *S. aureus* alpha haemolysin expression product and/or a specific detection molecule suitable for determining the level of alpha haemolysin, which is provided in the storage-stable form, preferably as a packaged unit.

Specifically the diagnostic kit is used in a method described herein, wherein the diagnostic kit is a diagnostic combination kit as further described herein.

Further described herein is a diagnostic kit for the determination of the alpha haemolysin level in a biological sample which comprises an alpha haemolysin specific detection molecule and an alpha haemolysin reference material, e.g. a standard alpha haemolysin preparation, to quantitate the alpha haemolysin level in said biological sample.

Specifically, described herein is a method of detecting the level of an alpha haemolysin in a biological sample suspected of containing the alpha haemolysin, which comprises
- providing a reaction mixture comprising a substrate, wherein the substrate comprises at least one alpha haemolysin binder;
- contacting the biological sample with the reaction mixture; and
- detecting a detection signal corresponding to the amount of alpha haemolysin recognized by the binder.

Specifically a negative control or reference control composition may be used in a kit as described herein. In such case, the detection signal may be normalized to a control signal corresponding to the amount of signal produced by the control composition, thereby detecting the presence or level of the alpha haemolysin in the biological sample. Normalizing the detection signal may comprise subtracting the control signal from the detection signal.

Further described herein is a diagnostic combination kit comprising the diagnostic kit for the determination of the alpha haemolysin level, and further comprising a kit for determining a further *S. aureus* marker.

Specifically, provided herein is a diagnostic combination kit, in particular to determine the alpha haemolysin level and at least one additional marker indicative of *S. aureus,* or *S. aureus* colonization or infection in the same or separate biological sample(s) of the same subject, comprising
a) a kit comprising one or more reagents capable of specifically reacting with the S. *aureus* alpha haemolysin expression product and/or a specific detection molecule suitable for determining the level of alpha haemolysin; and
b) a kit comprising a reagent and/or a specific detection molecule suitable for determining a gene expressing a *S. aureus* specific bacterial component or the respective gene expression product of a *S. aureus* specific bacterial component, other than alpha haemolysin, preferably selected from the group consisting of
   i) *S. aureus* Protein A;
   ii) a *S. aureus* antibiotic resistance marker preferably at least any of PBP2a, the *mecA* gene or an expression product of the *mecA* gene;
   iii) any other *S. aureus* antigen including a *S. aureus* cytotoxin, preferably PVL/LukSF, LukGH (LukAB), gamma-haemolysin, LukED; *S. aureus* virulence factors, secreted and surface proteins; or any *S. aureus* specific compounds such as lipoteichoic acid, and
   iii) a combination of any of the bacterial components of i), ii) and/or iii), preferably determining as additional markers at least *S. aureus* Protein A and PBP2a.

A MRSA marker, which is e.g. any of PBP2a, the *mecA* gene or an expression product of the *mecA* gene, would indicate the presence or absence of MRSA or MSSA, thus, further conclusions can be drawn to the prediction of disease or disease progression and the treatment regimen to be given to the patient.

*S. aureus* specific bacterial markers, which is e.g. *S. aureus* Protein A would indicate the presence or absence of *S. aureus,* thus, further conclusions can be drawn to the prediction of disease or disease progression and the treatment regimen to be given to the patient in the absence of other specific bacterial identification tests.

Specifically, the kit comprises one or more reagents for carrying out the method described herein.

### FIGURES

**Figure 1****. Hemolytic Phenotypes of *S*. *aureus* Isolates on Sheep-Blood Agar Plates.** *S*. *aureus* strains were plated on sheep-blood agar-plates and isolates were categorized into strong to weak or no beta-hemolysis (from 4+ to -), characteristic for alpha-hemolysin expression, according to the diameter of cleared halo around the colonies based on semi-quantitative evaluation. Turbid halos surrounding the colonies were accounted for beta-hemolysin (Hlb) expressing clones.

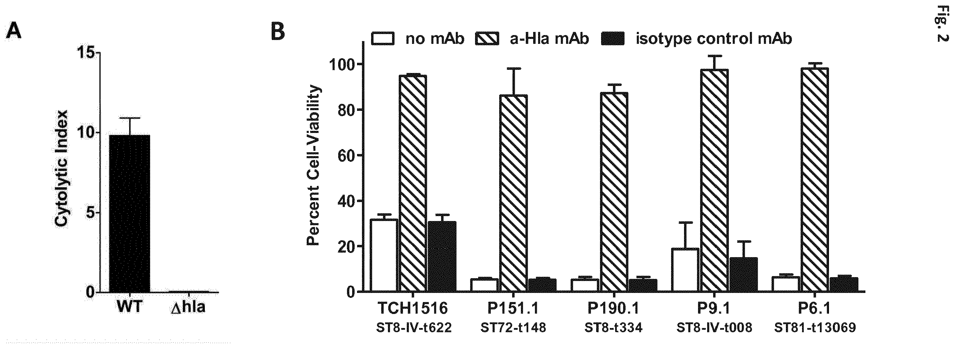

**Figure 2****. Cytolysis of Human Alveolar Epithelial Cells by *S*. *aureus* Culture Supernatants is Mediated by Alpha-Hemolysin.** A549 cells were incubated with culture-supernatants of *S. aureus* strains and cytotoxicity was measured as described in the methods. Mean values +/- SEM obtained with independent biological replicates of the same isolates are shown. **A:** USA300 TCH1516 wild-type and isogenic *Δhla* strain; **B:** Culture supernatants of four isolates with high Hla-activity were used for intoxication in the presence or absence of 66.7 nM of an Hla-neutralizing monoclonal antibody and a corresponding isotype control mAb. Percent cell-viability was determined compared to mock-treated cells.

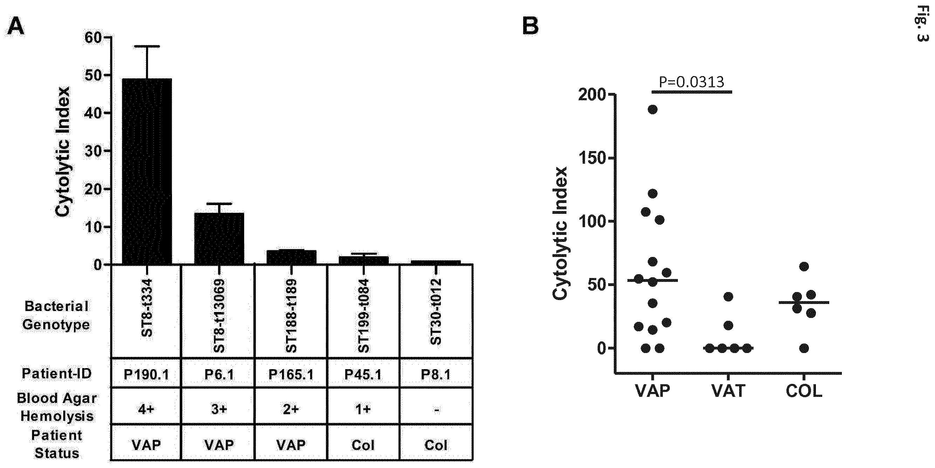

**Figure 3****: Hla-Induced Sheep-Blood Agar Hemolysis Profiles Correlate with the *In Vitro* Detectable Cytotoxicity towards Human Alveolar Epithelial Cells. A**: *S*. *aureus* isolates selected based on their hemolysis profiles on sheep-blood agar plates (Figure 1) were tested for their cytotoxicity towards A549 cells determined as described in the methods. **B.** Cytotoxicity indices of MSSA isolates are shown in different groups of patients as indicated. Each patient is represented by the first available isolate. Horizontal lines indicate group median values. Individual groups were statistically compared by Wilcoxon-Rank test (indicated as p-values).

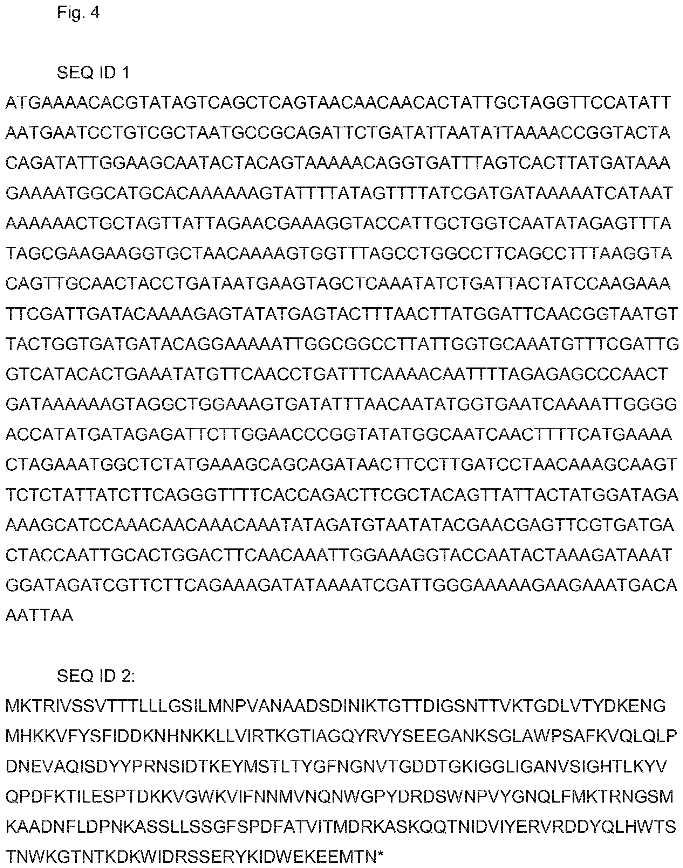

**Figure 4****: *S*. *aureus* toxin sequences:** representative alpha haemolysin sequences obtained from the USA300 TCH1516 strain (Genbank, accession number CP000730). Other strains may comprise one or more point mutations.
   SEQ ID 1 *hla* nucleotide sequence of the USA300 TCH1516 strain (Genbank, accession number CP000730)
   SEQ ID 2: Hla amino acid sequence of the USA300 TCH1516 strain

### DETAILED DESCRIPTION

The term "alpha haemolysin" as used herein shall refer to alpha-toxin of *S. aureus,* which is understood as the alpha-toxin protein (e.g., with the amino acid sequence as shown in Fig. 4, SEQ ID 2, or naturally-occurring or non-naturally-occurring or artificial variants thereof, identified by an amino acid sequence with at least 95% sequence identity), or diagnostically relevant parts or fragments of such protein, complexes of the alpha heamolysin with native or artificial reagents or reaction products, the *hla* gene (e.g., the nucleic acid as shown in Fig. 4, SEQ ID 1) or gene expression products which may be proteinaceous or genetic molecules, e.g. RNA, mRNA, or DNA, either single stranded or double stranded, sense or antisense nucleic acids or polynucleotides, or complementary nucleic acids or polynucleotides thereto. Nucleic acid molecules or polynucleotides specifically are determined in the isolated form. For example, isolated RNA molecules include *in vivo* or *in vitro* RNA transcripts of the DNA molecules.

The term "biological sample" as used herein shall refer to any material obtained from a subject, such as a human being, that contains, or potentially contains, biological material which could contain *S. aureus.* The biological sample can be a tissue, fluid or cell culture sample. Examples of samples for use in accordance with the invention include, but are not limited to patient samples, e.g., tissue or body fluids, specifically a respiratory tract specimen such as endotracheal aspirates, pleural fluid, lung tap, nasal swab or sputum, a blood sample, stool sample, skin and urine sample or cerebrospinal fluid.

The biological sample typically comprises a complex biological matrix such as complex viscous biological fluids containing multiple types of biological and small organic molecules, for example mucous exudates rich in protein matter. Suitable additives or extraction procedures may be used to reduce the non-specific binding that can be associated with a matrix in the sample and/or lower the matrix viscosity by solubilizing and/or breaking down viscous or solid components of the sample matrix. Sample preparation methods may be employed that liberate markers from organisms and/or break down and/or liquefy biological matrices. Biological matrices that may be analyzed include mucus-containing samples such as nasal secretions, sputum, phlegm, pharyngeal exudates, urethral or vaginal secretions, and washes of such membrane surfaces.

Suitable sample preparation methods include method steps to reduce the effect of the biological matrix on the assay. Such method steps may include but are not limited to, e.g., capture, chromatography, spin-centrifugation and dialysis.

The material obtained form a subject may also be in the form of bacterial isolates, e.g., in the form of a cell culture for cultivating the isolated *S. aureus* or a cell culture product. Culture media may be selective to enrich solely the *S. aureus* population, or non-selective.

Bacterial isolate prepration typically involves an incubating step to maintain the the sample in conditions that enhance the proliferation of *S. aureus,* thereby enriching the *S. aureus* population in the sample.

Once the isolate is obtained, the bacterium may be further investigated by biochemical and/or serological tests, e.g., to determine the MSSA or MRSA type, and the level of alpha haemolysin expressed. Several typing methods are available to study *S. aureus* strains. These methods typically include serotyping, toxin-typing, standard typing for genetic relationship/phylogeny including multi-locus sequence typing (MLST), spa-typing, Pulsed Field Gel Electrophoresis (PFGE). Other typing methods include gene-typing of virulence factors and other important staphylococcal factors by e.g. PCR, multiplex-PCR, microarrays, Northern/Southern blotting and protein based methods such as ELISA, Western-blot and mass spectroscopy. Typically, the antibiotic resistance profile is investigated using antibiograms.

The term "alpha haemolysin binder" as used herein shall refer to any useful binding agent of alpha haemolysin. In particular the alpha haemolysin binder recognizes the alpha haemolysin protein (Hla) or the *hla* gene or its expression product, e.g. peptide or polypeptide, RNA or DNA. It may bind directly or indirectly, such as binding to the alpha haemolysin which is present in the form of a complex of the alpha haemolysin and a reagent, either a native or an artificial reagent.

The binder can be directly labeled or the binding agent can be indirectly labeled. The indirect label may comprise a labeled binding agent that forms a complex with the binding agent to the alpha haemolysin.

In some aspects the binder or a detection molecule is conjugated to a detectable label which may include molecules that are themselves detectable (e.g., fluorescent moieties, electrochemical labels, metal chelates, etc.) as well as molecules that may be indirectly detected by production of a detectable reaction product (e.g., enzymes such as horseradish peroxidase, alkaline phosphatase, etc.) or by a specific binding molecule which itself may be detectable (e.g., biotin, digoxigenin, maltose, oligohistidine, 2,4-dinitrobenzene, phenylarsenate, ssDNA, dsDNA, etc.).

The term "alpha haemolysin binder" is specifically understood to encompass selective or specific alpha haemolysin reagents and/or detection molecules.

In one specific aspect, the binder is a detection molecule.

Specific alpha haemolysin binders or detection molecules may be produced synthetically or be of cellular or bacterial origin or present in a biological sample, e.g., isolated and partially or substantially purified. Such isolated components have typically been removed from its native environment. Specific alpha haemolysin binders are cells or cellular components that react with alpha haemolysin in a functional assay.

In one specific aspect, the binder recognizes alpha haemolysin in a reaction mixture. The resulting binder-alpha haemolysin complex can then be contacted with another detection molecule that binds to the complex. The detection molecule may bind to the complex by way of the binder and/or the alpha haemolysin.

Among the suitable alpha haemolysin binders are e.g., antibodies, antibody fragments, such as Fab, F(ab)2, F(ab)', Fv, scFv, or single chain antibodies, antibody-like scaffolds, where scaffolds are typically consisting of a constant part and a variable part that is complementary to the structure of the target antigen or an epitope thereof, e.g. aptamers. Further examples of alpha haemolysin binders are cellular receptors, such as those involved in disease pathogenesis, e.g., proteinaceous and non-proteinaceous toxin-specific receptors (e.g. ADAM-10), ligands, such as soluble ligands involved in disease pathogenesis, e.g. (shedded) membrane vesicles, nucleotide seqences specifically hybridizing to the alpha haemolysin gene or an expression product of said gene, such as oligonucleotides or probes, specifically including primer sequences to react with the expression product, or probes capturing the respective expression products.

The person skilled in the art can design primers for amplification of nucleic acid sequences specific to the alpha haemolysin from the published literature or from public databases. Nucleic acid molecules possibly used as detection molecules may be in the form of RNA, such as mRNA, or in the form of DNA, including, for instance, DNA and genomic DNA obtained by standard DNA-preparation methods, cloning or produced synthetically. The DNA or RNA may be double-stranded or single-stranded. Single-stranded DNA or RNA may be the coding strand, also known as the sense strand, or it may be the non-coding strand, also referred to as the anti-sense strand.

The practice of the present invention employs, unless otherwise indicated, conventional techniques of immunology, biochemistry, chemistry, molecular biology, microbiology, cell biology, genomics and recombinant DNA, which are within the skill of the art. See e.g. Sambrook, Fritsch and Maniatis, MOLECULAR CLONING: A LABORATORY MANUAL, 2nd edition (1989).

The detection of only specifically hybridizing sequences will usually require stringent hybridization and washing conditions such as 0.1 x SSC, 0.1% SDS at 65°C. As is well-known in the art, the length of the nucleotide sequence and the composition of the nucleic acid to be determined constitute further parameters of the hybridization conditions.

"Specific binding" or "selective binding" as used herein, means that the binder, e.g. antibody or antigen-binding portion thereof, exhibits appreciable affinity for the alpha haemolysin or a respective epitope or nucleotide sequence in a heterogeneous population of molecules. Thus, under designated conditions (e.g., immunoassay or hybridization conditions), a binder specifically binds to the target alpha haemolysin and does not bind in a significant amount to other molecules present in a sample. The specific binding means that binding is selective in terms of target identity, high, medium or low binding affinity or avidity, as selected. Selective binding is usually achieved if the binding constant or binding dynamics is at least 10-fold different (understood as at least 1 log difference), preferably the difference is at least 100-fold (understood as at least 2 logs difference), and more preferred a least 1000-fold (understood as at least 3 logs difference) as compared to another target.

The term "specificity" or "specific binding" is also understood to apply to binders which bind to one or more molecules, e.g. cross-specific binders. Preferred cross-specific (also called polyspecific or cross-reactive) binders targeting at least two different targets or epitopes or nucleotide sequences of such targets or targeting a cross-reactive epitope or nucleotide sequence on at least two different targets, specifically bind the targets with substantially similar binding affinity, e.g. with less than 100-fold difference or even less than 10-fold difference.

The term "detection molecule" as used herein shall refer to a molecule or a complex of molecules that is detectable through a specific characteristic or a label, e.g. upon a specific binding reaction to alpha haemolysin. Such binding reaction by the detection molecule can be directly and immediate, or mediated by a separate alpha haemolysin binder. The label is typically a molecule or part of a molecule that can be detected in an assay. Examplary labels are chromophores, fluorochromes, or radioactive molecules.

The detection molecule may be a specific alpha haemolysin binder as such, or a molecule detecting the alpha haemolysin through its binding reaction with an alpha haemolysin binder, e.g., capturing the alpha haemolysin, or the respective binder, or the alpha haemolysin-binder complex. A detection molecule can be both in one molecule, the alpha haemolysin binder and the dectection molecule. The label may be part of the alpha haemolysin binder or used as a separate label or on a detection molecule separate from the alpha haemolysin binder.

Preferred detection molecules are specific alpha haemolysin binders which are labeled by an endogenous label or by a separate label that is e.g. conjugated to the molecule.

The term "diagnostic kit" as used herein refers to a kit or set of parts, which in combination or mixture can be used to carry out the measurement/detection of one or more analytes or markers to determine a disease or disease condition, or to predict the disease and particularly the onset of disease, or the disease progression. In particular, the kit contains at least a detection molecule and/or a binder, wherein the detection molecule and/or the binder specifically recognizes the analyte or marker, or a reaction product of such analyte or marker. In addition, various reagents or tools may be included in the kit. The diagnostic kit may comprise any useful reagents for carrying out the subject methods, including substrates such as microbeads or planar arrays or wells, reagents for biomarker isolation, detection molecules directed to specific targets, reagents such as primers for nucleic acid sequencing or amplification, arrays for nucleic acid hybridization, detectable labels, solvents or buffers and the like, various linkers, various assay components, blockers, and the like.

A kit may also include instructions for use in a diagnostic method. Such instructions can be, for example, provided on a device included in the kit, e.g. tools or a device to prepare a biological sample for diagnostic purposes, such as separating a cell and/or protein containing fraction before determining a marker. The kit may conveniently be provided in the storage stable form, such as a commercial kit with a shelf-life of at least 6 months.

Specific diagnostic kits also comprise a solid support comprising a detection molecule or having an immobilized patterned array of detection molecules directed against markers of interest, preferably including a first region containing a first binding reagent directed against a first marker and a second region containing a second binding reagent directed against a second marker.

In particular, a sandwich format can be used. For example, one or more binder is conjugated to a substrate prior to the contacting with a biological sample. The one or more binder may be conjugated to a detectable label to serve as a detection molecule. According to a specific aspect, the one or more binder is conjugated to a detectable label. In this configuration, the one or more binders may be conjugated to a substrate prior to the contacting with the biological sample to serve as a capture agent. Furthermore, the one or more binder can be conjugated to a substrate prior to the contacting with the biological sample, and/or the one or more binder is conjugated to a detectable label. In such cases, the one or more binder can act as either or both of a capture agent and a detection agent.

If more than one analyte or marker is detected, the kit may be provided as a diagnostic combination kit, which is understood to combine the reagents and tools suitable for measuring all analytes or markers in one kit. Such combination kit may be composed of more than one part, wherein a first part may include a detection molecule suitable for measuring a first analyte or marker, and additional part(s) which may include a detection molecule suitable for measuring additional analytes or markers.

The diagnostic kit is specifically provided for use in an immunoassay, wherein the detection molecule is a specific binder that binds to the analyte or marker by an immunoreaction. Such binder may be antibodies or antibody fragments or antibody-like scaffolds binding to a target antigen.

Suitable immunoassays are any of ELISA, CIA, RIA, IRMA, agglutination assay, immunochromatography, lateral flow immunochromatographic assay (such as dipstick assays) and Western-blot. Commercial agglutination assays are available for example for overnight culture samples to identify *S. aureus* via presence specific *S. aureus* surface antigens include detection of Clumping factor or Protein A (e.g. Dry Spot Staphytect Plus, Oxoid; Pastorex Staph Plus test, Bio-Rad; Slidex Staph-Kit and Slidex Staph Plus test, bioMérieux) and MRSA specific protein PBP2a (e.g. MRSA latex test, Hardy Diagnostics; MRSA screen test, Denka Seken Co. Ltd).

It is evident for the skilled person that such assays and test system for the measurement for alpha haemolysin levels can easily be obtained or generated. The corresponding test systems and assays need corresponding adjustments and evaluation in order to standardize the test for the measurement of elevated alpha haemolysin levels.

Specifically, when the target analyte comprises a protein or polypeptide, measuring the target may comprise use of mass spectrometry-based screening methods or mass spectrometry (MS), peptide mass fingerprinting (PMF; protein fingerprinting), sequencing, N-terminal amino acid analysis, C-terminal amino acid analysis, Edman degradation, chromatography, electrophoresis, (two- dimensional) gel electrophoresis (2D gels), NMR, antibody array, and immunoassay.

Specific diagnostic kits are provided for use in a molecular genetics assay which is suitable for determining the level of relevant oligo or polynucleotides or nucleic acids. According to a specific aspect, the amplification of nucleic acid sequences corresponding or specific to the target analyte or marker is by gene-chip microarray or PCR. According to a different aspect, the amplification is by RNA RT-PCR or by immunoPCR. According to a different aspect, the amplification is by cascade amplification. According to yet a different aspect, the amplification is by ligase chain reaction.

In a specific aspect, the detection of the amplification product is by gel electrophoresis, such as agarose gel electrophoresis. In another aspect, the detection of the amplification product is by hybridization with a nucleic acid probe or other probes. The hybridization can be solid phase or liquid phase. The hybridization can be dot hybridization with a nucleic acid probe or other probes. The dot hybridization can be a radioactive dot blot or non-radioactive dot blot. The dot hybridization can be a dot blot micromethod or another method.

In another specific aspect, the detection of the amplification product is by real time fluorescence measurement. In another specific aspect, the detection of the amplification product is by immunoenzymatic assay using anti-RNA:DNA hybrid antibodies. In another specific aspect, the detection of the amplification product is by nucleic acid probe assay using a fluorescein dye and/or fluorescent dye photometry. In another specific aspect, the detection of the amplification product is by use of immobilized nucleic acid probes. Examples of substrates upon which the nucleic acid probes can be immobilized comprise a microplate, a membrane filter, a strip, a tube or a microchip. According to one specific aspect, a DNA probe is bound to a solid phase or carrier. A labeled amplification product is then allowed to bind the DNA probe. The amplification product can be labeled, for example, with biotin, a colorimetric probe or a fluorescent dye. The presence of amplification product can then be detected by detection of the label, or where applicable, by detection of a secondary label that binds to or reacts with the labeled amplification product, such as horseradish peroxidase, alkaline phosphatase, labeled anti-biotin antibody (streptavidin), avidin, avidin-biotin complex or avidin-streptavidin complex.

The term "level" in relation to a marker or the alpha haemolysin marker is herein understood as the level of such marker in a biological sample which is greater than a reference which is conveniently a standard level, i.e. the level produced by a standard, or reference control level, i.e. a level produced by a reference control, such reference may be a cutoff value of the marker.

The elevated level is e.g., significantly higher than the reference. The term "significant" with respect to the overexpression of the alpha haemolysin marker as used herein shall refer to at least a two-fold higher amount of the standard deviation, preferably at least a three-fold difference. In a preferred quantitative determination method, the expression of the marker is normalized to the median expression of one or more reference genes, e.g. used as internal control.

With respect to a specific reference value, such as derived from a standard, training data or threshold, a significant elevated or increased amount is understood to refer to an at least 1.5-fold higher amount, preferably at least 2- or 3-fold difference.

As used herein, the term "cutoff value" refers to a threshold value which distinguishes subjects who are suffering from a disease or disease condition from patients and/or subjects who are not suffering from the disease or disease condition, and in particular distinguishes subjects who are at high risk of disease or disease progression from patients and/or subjects who are not at such high risk of disease or disease progression.

In the context of predicting the *S. aureus* disease or the onset of disease in a subject heavily colonized by *S.aureus*, an elevated level of alpha haemolysin is typically greater than a cutoff value and a non-elevated level of alpha haemolysin is less than or equal to the cutoff value, which is e.g. the value reflecting the alpha haemolysin expressed by a non-hemolytic strain (which includes also low-expressors) in a comparable setting.

As used herein, the terms "reference" or "control" may be an agent or value that allows comparison to the result of such diagnostic assays so that conclusions may reasonably be drawn based on differences or similarities observed. Suitable values are e.g. predetermined levels of predictability for disease or disease progression, such as obtained from biological sampels of a patient that later developed severe symptoms of disease or disease progression. In this regard, positive predictive values or negative predictive values may be used as a reference.

The reference or control as described herein specifically refers to the level and/or concentration of alpha haemolysin in a biological sample from a patient colonized by *S. aureus,* in specific cases MSSA, who is asymptomatic but heavily colonized with *S. aureus* (and may have experienced prior to administration of an antibiotic treatment), such patient is determined not to develop symptomatic disease, and in particular VAT or VAP. Such determination of a reference or control may include evaluation of a series of samples and the patient's history to determine which the appropriate reference values are. A control may conveniently be an internal control or external control.

The levels may be determined in quantitative or semi-quantitative assays. Such assays typically have read-outs which are concentrations (w/w) or (w/v) or else a value indicating the results compared to a reference, such as a percentage, or "+", "++", or "+++", the higher number of "+" indicating the higher values.

Further described herein are functional assays to measure the alpha haemolysin level. Such assays could involve measuring the hemolysis pattern of *S. aureus* isolates cultured on sheep blood agar plates using standard microbiology methods, with high beta hemolysis pattern (2+, 3+ or 4+) indicating high Hla activity compared to no or low (1+) beta hemolysis on the sheep blood agar plate.

The determination of the alpha haemolysin level is typically not by a qualitative assay only, because the detection of presence or absence of the alpha haemolysin gene without determining its expression level, would not be predictive of the disease or disease progression. Thus, a mere assay for determining solely the presence of the *hla* gene in the biological sample or in the *S. aureus* isolate would not be sufficient for predicting the patient's risk of disease or disease progression. However, the expression or over-expression of the gene-product (i.e. the expression which is higher than a reference) would result in an alpha haemolysin protein level which can be determined by a quantitative or semi-quantitative method and which is indicative of such prediction and therefore be predictive of disease onset in a heavily *S. aureus* colonized patient.

The term "marker" herein also termed "biomarker" shall refer to a biological indicator that reflects disease status or development, but may not necessarily be involved in the disease process itself. In some early stage *S. aureus* patients, antibacterial therapy can be highly effective, providing a significantly improved cure of disease. Research efforts are being made to identify and validate markers that can help identify subsets of patients that will benefit most from such therapy. In addition to the advantage of such predictive markers, markers are playing an important role in the development, clinical evaluation and monitoring of *S. aureus* therapies. Markers can aid patient selection by allowing stratification and risk assessment and can potentially predict treatment responses based on the presence or extent of such markers. The present alpha haemolysin marker may advantageously be used to identify patients at risk as early as possible.

The present alpha haemolysin marker can be used as a predictive marker, e.g. as a stand-alone diagnostic or in combination with further diagnostic measures, such as the determination of further markers (e.g. in a diagnostic combination kit).

In particular, the present alpha haemolysin marker may be used for monitoring or surveillance. For example, the alpha haemolysin marker can be used for surveillance purposes supporting the physician predict or monitor the progression of disease.

The alpha haemolysin marker can also be used in a diagnostic combination kit employing determination of the alpha haemolysin marker and a further *S. aureus* marker, such as Pbp2a and/or SpA, which is provided as stand-alone diagnostic (i.e. which provides a diagnosis without further diagnostic measures).

The higher the alpha haemolysin level in the test sample, the higher the likelihood for a fast onset of the disease. It was, for example, surprisingly found that a higher alpha haemolysin expression (compared to baseline values) is clearly correlated with a more rapid onset of VAP or VAT. Another important aspect described herein is that the correlation of alpha haemolysin level and the onset of disease appears to be highly significant in those patients which are colonized by MSSA. Thus, the method described herein is specifically applied to those patients where the *S. aureus* clones are (pre)determined to be MSSA, or which show further indicators or markers of MSSA or the absence of MRSA indicators or markers.

The term "onset of disease" is specifically understood as the timepoint when a subject develops symptoms of a disease leading to a diagnosis. By predicting the onset of disease, a risk determination is provided which provides for the ability of the skilled person to identify a subject that is susceptible to the disease with a high risk, such that the subject may receive a respective therapy even before the disease is diagnosed.

The marker may be directly determined as an analyte targeted by an assay, which may determine the marker level. The alpha haemolysin is specifically understood as an *in vitro* marker of *S. aureus* disease onset, specifically in patients that are colonized by MSSA. Such an *in vitro* marker is conveniently determined by an *in vitro* method, e.g. in a biological sample of the patient *ex vivo.*

Besides the alpha haemolysin marker, there may be other markers of *S. aureus* or the associated disease onset or characterization of the disease to aid treatment which are further determined by a qualitative, semi-quantitative or quantitative assay, e.g. by a diagnostic combination method or kit. Among the other markes there are virulence factors, such as exotoxins, endotoxins or surface proteins, antibiotic resistance markers, such as methicillin encoded by the SCCmec, other resistance genes that dominantly came from (horizontal) gene transfer of mobile genetic elements e.g. beta-lactamase, kanamycin-resistance, erythromycin-resistance, etc., specific bacterial components, non-proteinaceous factors such as WTA and LTA, capsular polysaccharides, genes expressing any of cytotoxin, virulence factors, secreted and surface proteins, or respective gene expression products.

The virulence of *S. aureus* is due to a combination of numerous virulence factors, which include surface-associated proteins that allow the bacterium to adhere to eukaryotic cell membranes, capsular polysaccharide and surface protein A (SpA) that protects it from opsonophagocytosis, and several exotoxins. *S. aureus* causes disease mainly through the production of secreted virulence factors such as hemolysins, enterotoxins and toxic shock syndrome toxin. These (secreted) virulence factors suppress the immune response by inactivating many cellular and humoral immunological mechanisms in the host, and cause cell and tissue destruction and help establish the infection. The latter is for example accomplished by a group of pore forming toxins, the most prominent of which is Hla.

*S. aureus* produces a diverse array of further virulence factors and toxins that enable this bacterium to neutralize and withstand attacks by different kinds of immune cells, specifically subpopulations of white blood cells that make up the body's primary defense system. The production of these virulence factors and toxins allows *S. aureus* to maintain a pathogenic state. Among these virulence factors, *S. aureus* produces several bi-component leukotoxins, which damage membranes of host defense cells and erythrocytes (e.g. for nutrient acquisition) by the synergistic action of two non-associated proteins or subunits. Among these bi-component toxins, gamma-hemolysin (HlgAB and HlgCB), LukGH (LukAB) and LukSF/PVL are the best characterized.

The toxicity of the leukocidins towards mammalian cells involves the action of two components. The first subunit is named class S component, and the second subunit is named class F component. The S and F subunits act synergistically to form pores on e.g. white blood cells including monocytes, macrophages, dendritic cells and neutrophils (collectively known as phagocytes). The repertoire of bi-component leukotoxins produced by *S. aureus* is known to include cognate and non-cognate pairs of the F and S components, e.g. gamma-hemolysins, PVL toxins and PVL-like toxins, including HlgAB, HlgCB, LukSF, LukED, LukGH, LukS-HlgB, LukSD, HlgA-LukD, HlgA-LukF, LukG-HlgA, LukEF, LukE-HlgB, HlgC-LukD or HlgC-LukF.

According to a specific aspect, the alpha haemolysin level is determined, and in addition another marker indicative of MSSA or indicative of the absence of MRSA is determined. For example, the other marker could be penicillin-binding protein 2a (PBP2a), or its cognate gene (*mecA*) or an expression product of the *mecA* gene, which is a determinant for MRSA. The absence of such other markers would indicate that the patient is colonized by MSSA. Therefore, if the alpha haemolysin level is measured as an indicator of disease onset and the other marker is indicating that the *S. aureus* is MSSA, the antibacterial therapy would be strongly indicated. A further specific aspect includes separate or parallel detection of a specific *S. aureus* marker, for example *S. aureus* Protein A to confirm that the colonizing bacterium in the biological sample is *S. aureus.* Although alpha haemolysin itself is a *S. aureus* specific protein, inclusion of a further *S. aureus* marker such as *S. aureus* Protein A would act as a 'positive control' or 'alternative marker' to confirm the presence of *S. aureus* as the colonizing bacterium in the absence of a positive signal for alpha haemolysin.

The term *"S. aureus* infection" is understood in the following way: *Staphylococcus aureus* is a Gram-positive, coccal bacterium that is a member of the Firmicutes, and is frequently found in the human respiratory tract and on the skin. It is positive for catalase and nitrate reduction. Although asymptomatic nasal colonization with *S. aureus* is common, the carriage of *S. aureus* is an important determinant of nosocomial infection. Spread of *S. aureus* (including MSSA and MRSA) generally is through human-to-human contact. Transmission of the pathogen is facilitated in medical settings where either healthcare worker hygiene or disinfection/decontamination strategies are insufficient. Introduction of the bacteria into the bloodstream can lead to various complications, including, but not limited to, endocarditis, meningitis, and if it is widespread, sepsis.

VAT is defined as an acute infection of the tracheobronchial tree without involvement of pulmonary parenchyma in a patient intubated and mechanically ventilated for ≥ 48 hours. Two conditions must be present: (i) Positive ETA cultures for ≥10⁵ CFU/mL or moderate or heavy (+++ (3+) or ++++ (4+)) bacterial growth of *S. aureus* and (ii) at least two of the following clinical signs and symptoms of infection: leukocytosis, fever or hypothermia, and purulent respiratory secretions.

VAP represents an acute infection of the pulmonary parenchyma in a patient intubated and mechanically ventilated for a minimum of 48 hours. Three conditions must be present: (i) Positive ETA cultures for ≥10⁵ CFU/mL or moderate or heavy (+++ (3+) or ++++ (4+)) bacterial growth of *S. aureus,* (ii) at least two of the following clinical signs and symptoms of infection: leukocytosis, fever or hypothermia, and purulent respiratory secretions, and (iii) presence of a new and progressive infiltrate on a chest radiograph or CT scan.

*S. aureus* disease is specifically understood as a disease caused by *S. aureus* infection. In addition to VAT and VAP, such diseases include local and systemic disease. Severe cases of disease are e.g. bacteremia, sepsis and septic shock, pneumonia and chronic wound infection.

In general, bacteremias caused by *S. aureus* may be successfully treated with known conventional antibacterial therapy, such as treatment with antibiotics, steroid and non-steroid inhibitors of inflammation, and/or immunotherapy, e.g. employing antibodies specifically recognizing one or more *S. aureus* toxins, anti-bacterial or anti-inflammatory agents. Exemplary antibiotics used for treating patients with *S. aureus* infection are tigecycline, linezolid, methicillin and/or vancomycin.

However, with the recent emergence of strains of antibiotic-resistant bacteria, treating bacteremias of this nature has become significantly more difficult. Patients who develop *S. aureus* disease have longer hospital and ICU stays, high mortality, and greater health care costs than patients without *S. aureus* disease. Patient care may be improved and nosocomial infections may be reduced by preventing, rather than treating, *S. aureus* disease prophylaxis when a patient is heavily colonized by *S. aureus.*

The term "subject" as used herein shall refer to a warm-blooded mammalian, particularly a human being or a non-human animal. *S. aureus* is a critically important human pathogen that is also an emerging concern in veterinary medicine. It is present in a wide range of non-human animal species. Thus, the term "subject" may also particularly refer to animals including dogs, cats, rabbits, horses, cattle, pigs and poultry. In particular the medical use described herein or the respective method of treatment applies to a subject in need of prophylaxis or treatment of a disease condition associated with a *S. aureus* infection. The subject may be a patient at risk of a *S. aureus* infection or suffering from disease, including early stage or late stage disease. The term "patient" includes human and other mammalian subjects that receive either prophylactic or therapeutic treatment. The term "treatment" is thus meant to include both prophylactic and therapeutic treatment.

A subject is e.g. treated for prophylaxis or therapy of *S. aureus* disease conditions. In particular, the subject is treated, which is either at risk of infection or developing such disease or disease recurrence, or a subject that is suffering from such infection and/ or disease associated with such infection.

Specifically the term "prophylaxis" refers to preventive measures which is intended to encompass prevention of the onset of pathogenesis or prophylactic measures to reduce the risk of pathogenesis.

Specifically, the treatment may be by interfering with the pathogenesis of *S. aureus* as causal agent of the condition, wherein the pathogenesis includes a step of forming a pore on the subject's cellular membrane, e.g. by the specific virulence factors or toxins.

Therefore, provided herein is a method and diagnostic tool for practitioners to determine whether a heavily colonized patient is likely to develop *S. aureus* infection and diseases caused by colonizing bacteria is fast or slow or whether *S. aureus* colonization has the potential of a fast or slow disease onset. The alpha haemolysin level of biological samples and *S. aureus* isolates turned out to be surprisingly predictive for virulence. This is of particular importance since the clinical avenues to be taken in order to ameliorate the medical condition of a patient colonized by *S. aureus* (human as well as non-human patients) depends on the prediction how the disease will develop or progress, as early as possible. In short, provided herein is the teaching that a clear and strong correlation between the alpha haemolysin level (and/or indicators of MSSA) and the onset of disease caused by *S. aureus* can be drawn.

It turned out that *S. aureus* isolates from endotracheal aspirates (ETA) obtained by serial sampling of ventilated patients heavily colonized with *S. aureus* could be indicative of onset of VAP. According to an example, serial *S. aureus* ETA isolates from ventilated patients were analyzed and alpha haemolysin activity was determined by semi-quantitative analysis of hemolysis on blood-agar and quantitative measurement of cytolysis of human lung epithelial cells.

The majority of VAP cases were caused by MSSA, and MRSA isolates were mainly recovered from colonized patients. High alpha haemolysin activity in MSSA, but not MRSA, isolates was a marker for the *S. aureus* disease or disease progression to or the presence of VAP. The sheep blood agar hemolysis test, is a simple assay that can be performed in routine microbiological laboratories to measure alpha haemolysin activity and serve as predictor for VAP in ventilated patients colonized with MSSA isolates. These findings may expedite (i) prevention of VAT or VAP using prophylactic measure or (ii) initiation of earlier therapy on the onset of symptoms and therefore disease onset and earlier use of antibiotic therapy that both may translate into improved patient outcomes, such as overall survival.

Airway colonization by MSSA strains with high alpha haemolysin production is a valuable predictor for VAP. The present invention may help differential diagnosis and patient management, e.g., earlier use of targeted (narrow spectrum) antibiotic therapy.

The methods and diagnostic kits described herein may be particularly useful for predicting and/or monitoring the *S. aureus* disease, e.g., by determining the alpha haemolysin level in a series of biological samples from a patient. An increase of the alpha haemolysin level over time would indicate the increase of the risk of disease or disease progression.

The present invention is further illustrated by the following examples without being limited thereto.

### EXAMPLES

### Example 1: Alpha haemolvsin activity of methicillin-susceptible S. aureus predicts ventilator-associated pneumonia

### Generation of the Isogenic S. aureus Gene-Deletion Mutant Lacking hla-Expression

The isogenic *S*. *aureus hla* gene-deletion mutant was generated in the sequenced, USA300 CA-MRSA strain TCH1516 (ATCC® BAA1717™, LGC Standards, Teddington, UK) with homologous recombination based on previously published methods [15] using the primer-pairs listed in Table 1 and the pKFT gene-deletion vector, a previously reported shuttle-vector for *E. coli* and *S*. *aureus* [16].

**Table 1: Primers used (enzymatic restriction sites: underlined; fusion overlaps: italic)**

| Purpose | Locus/Type | Primer | Nucleotide Sequence (5' - 3') |
|---|---|---|---|
| gene-typing | hla | LST_0001 | CTGTCGCTAATGCCGCAGATTC (SEQ ID 3) |
| | | LST_0002 | GAAGTCCAGTGCAATTGGTAGTC (SEQ ID 4) |
| gene-deletion | hla | ko-LST_0041 | CCGCGGATCCCTATTAGATATTTCTATGTAATGGC (SEQ ID 5) |
| | | ko-LST_0042 | *CCCATCCACTAAACTTAAACA*CATCATCCTTCTATTTTTTAAAACG (SEQ ID 6) |
| | | ko-LST_0043 | *TGTTTAAGTTTAGTGGATGGG*GTAAATTATTTGTTCATGTACAAA (SEQ ID 7) |
| | | ko-LST_0044 | TGGCACCCGGGCGTTAATTTCCCAATCATTAAAAACC (SEQ ID 8) |

### Blood Agar Hemolysis Test

S. *aureus* isolates were incubated for 16 h at 37°C on Columbia agar containing 5% sheep-blood (COS, bioMerieux, Marcy-l'Etoile, France). The hemolysis profiles were evaluated after a subsequent incubation for 24 h at 4°C. This test was performed with three different clones of the same isolate and results evaluated by three different individuals.

### Evaluation of the Cytotoxicity of S. aureus Culture Supernatants Using a Human Alveolar Epithelial Cell Line, A549

Cytotoxicity assays were performed using a human alveolar epithelial cell line (A549 ATCC® CCL-185™, LGC Standards, Teddington, UK) and bacterial culture supernatants of bacteria grown overnight in tryptic soy broth (TSB, Sigma-Aldrich, Steinheim, Germany).

Cells were grown as adherent cultures in Ham's F12-K medium (Gibco®, LifeTechnologies, Paisley, UK) supplemented with 1x penicillin-streptomycin solution (10x stock, PAA Laboratories, Pasching, Austria) and 10% fetal calf serum (FCS, Sigma-Aldrich) in 175 cm² tissue culture treated cell culture flasks. For the cytotoxicity assays, 2x10⁴ cells were seeded in cell culture medium, into 96-well half-area plates 16 h prior to the experiment. On the day of the experiment, the cell culture medium was removed and 50 µl of bacterial culture-supernatants (serial dilutions in TSB) were added. Cells were incubated (3 h, 37°C, 5% CO₂) prior removal of the medium, washing with 100 µl cell culture medium and incubation (3 h, 37°C, 5% CO₂) in 50 µl fresh cell culture medium (F12-K medium+ 5% FCS). Cell viability was measured (Cell Titer-Glo® Luminescent Cell Viability Assay Kit, Promega, Madison, WI, USA) at 590/35 nm in a microplate reader (Synergy HT, BioTek, Bad Friedrichshall, Germany).
Assay data are presented as "Cytotolytic Index" being the EC50-values representing the fold-dilution of culture-supernatant needed to reach half-maximal cytolysis, calculated based on the determined titration-curves using the Prism 6 (GraphPad, La Jolla, CA, USA) software-package. Statistical analyses were performed using the Prism 6 software-package. Kruskal-Wallis (non-parametric) test was applied as a global test for significance. Individual groups were statistically compared by Wilcoxon signed rank test if P<0.05 was obtained by the global test (indicated as p-values).
For Hla-inhibition experiments, bacterial culture-supernatants were pre-incubated with a constant concentration (10 µg/ml; 66.67 nM) of a single-specific, monoclonal anti-Hla antibody (Arsanis Biosciences, Vienna, Austria; alternatively, any specific anti-alpha haemolysin monoclonal antibody or a commercially available anti-haemolysin antibody can be used such as available from IBT, catalogue number 04-0010) prior to incubation with cells. Percent cell-viability was calculated relative to mock-treated cells, with recombinant Hla and an isotype-matched control mAb being included as positive and negative assay controls, respectively.

### Sheep-Blood Agar Hemolysis Patterns

Upon culturing the *S. aureus* isolates on sheep-blood agar, we detected a broad range of hemolytic activities (Figure 1). The majority of isolates displayed a hemolysis pattern characteristic for alpha-hemolysin (Hla) causing beta-hemolysis and a clear halo on sheep-blood agar plates. A few isolates showed hemolysis with a turbid halo, which is typical for beta-hemolysin (Hlb) production of *S. aureus.* The presence of the functional *hlb* gene in these isolates was confirmed by PCR (data not shown). The reported sheep-blood agar hemolysis test, is a simple assay that can be performed in routine microbiological laboratories to measure alpha-hemolysin activity.

### Hla-Activity in S. aureus Culture-Supernatants can be Specifically Determined with a Cytotoxicity Assay using a Human Lung Epithelial Cell Line

To determine the *in* vitro activity of Hla, we evaluated the specificity of the A549 cytotoxicity assay for Hla. This was demonstrated in two ways: (i) By the lack of cell lysis by a *hla* gene-deletion mutant *S*. *aureus strain* (TCH1516, USA300) (Figure 2A) and *(ii)* in the presence of an Hla-neutralizing monoclonal antibody using culture-supernatants of different, strongly beta-hemolytic *S. aureus* clones as determined by the sheep-blood agar assay (Figure 2B).

These data reflect the specificity of the A549 cytotoxicity towards Hla-activity which can therefore support the evaluation of Hla-activity as a biomarker for Hla-induced virulence of *S. aureus* isolates.

### Hla-Activity of S. aureus can be Measured and Correlated by both, the Sheep-Blood Agar Hemolysis Assay and the A549 Cytotoxicity Assay

To correlate the beta-hemolytic profiles on sheep-blood agar with alpha-hemolysin activity, we determined the cytolytic capacity of serially diluted overnight culture supernatants from *S. aureus* isolates using the human lung alveolar epithelial cell line (A549).

Similarly to blood agar hemolysis, a great variation in alpha-hemolysin activity measured in the A549 viability-assays was observed among the *S. aureus* isolates, importantly, with good correlation between the two assays (Figure 3A).

MSSA strains, obtained from VAP patients, were found to be more cytotoxic than those obtained from VAT (P=0.0313) or colonized patients (Figure 3B) based on group median values without reaching statistical significance for the latter if evaluated by non-parametric Kruskal-Wallis test probably due to low sample numbers.

These results provide evidence that carriage of highly hemolytic strains is a strong correlator for VAP.

The diagnosis of VAP is not clearly differentiated from VAT. Especially VAP diagnosis (diagnosis of a new or progressive infiltrate on chest radiograph in addition to VAT) can be difficult and sometimes needs interpretation of chest radiographs by several specialists to find a consolidated diagnosis. Further there are regional differences in providing data for the respective VAP or VAT diagnosis. Therefore, the present disclosure refers to VAP and/or VAT.

The data further support the finding that the activity of Hla *(i)* can be determined by sheep-blood agar hemolysis as well as the A549 lung epithelial cell assay with good correlation and *(ii)* serves as a potent predictor for VAP in ventilated patients colonized with MSSA isolates. The present results provide evidence that enhanced disease (e.g. upon heavy colonization) is strongly associated with highly hemolytic MSSA strains. In certain cases, MRSA strains can also express high levels of toxin(s). Therefore, the present results would further support the correlation with highly hemolytic MRSA strains.

These findings may expedite initiation of earlier therapy to improve patient outcomes through earlier use of targeted antibiotic therapy that may translate into improved patient outcomes, such as decreased ventilator days and length of ICU stay.

Detectable, heavy airway colonization is a prerequisite for development of disease. Therefore even asymptomatic patients who do not manifest the corresponding symptoms, yet can be at high risk for developing such symptoms and disease. Such risk can be detected at an early time-point which significantly supports an early diagnosis and initiation of treatment.

### Example 2: Biological sample preparation

Prior assessment of sputum or endotracheal aspirate samples obtained from patients, samples are stabilized in a buffer containing N-acetylcystein to reduce the mucoid. Human blood and serum samples are obtained by standard blood withdrawal procedures in sterile collection tubes optionally supplemented with anticoagulants such as Li-heparin or K₂-EDTA. Urine and stool samples can be used directly as they are taken from the patient. For the detection of alpha-hemolysin, samples may be diluted in buffers suitable for the downstream application such as protein detection and/or nucleotide detection methods.

### Example 3: Alpha-haemolvsin assays

### ELISA based detection of the alpha-hemolysin protein

This procedure exemplifies the ELISA based detection of the alpha-hemolysin as protein (Hla) from bacterial samples such as culture (supernatants) and/or human biological samples such as endotracheal aspirates.

For the detection of Hla, appropriate flat-bottom 96-well plates (e.g. MaxiSorp®, Nunc) are coated with serial fold-dilutions of the corresponding sample material and purified, recombinantly expressed Hla (for quantification) in duplicates or triplicates. Dilutions are prepared in coating buffer such as phosphate-buffered saline (PBS). Plates are incubated at 4°C for approximately 16 h to allow optimal coating efficiency. After removing the coating solutions, plates are blocked by adding blocking-solution (e.g. 200 ul 2% bovine serum albumin (BSA) in PBS) and incubation at room-temperature for at least 1 h. Following three washing cycles (e.g. three-times 200 µl of 0.005% Tween in PBS) plates are incubated at room-temperature for 1h with an anti-Hla mAb dilution (e.g. 100 µl mAb diluted at 1 µg/ml in 1% BSA supplemented with 0.05% Tween). Following three washing cylces (same procedure as above) a secondary detection antibody (species-matched for the constant-domain of the anti-Hla mAb (for example, any specific anti-alpha haemolysin monoclonal antibody or a commercially available anti-haemolysin antibody available such as available from IBT, catalogue number 04-0010) and e.g. conjugated with horseradish peroxidase (HRP) or a fluorochrome; e.g. 100 µl of a 1:5000 dilution in 1% BSA supplemented with 0.05% Tween) is used for incubation at room-temperature for 1 h. Following another washing cycle (same procedure as described above), either (i) developing solution (e.g. ABTS for enzymatic reaction with HRP) is added and incubated at room-temperature for 20min in the dark and the reaction stopped with sulfuric acid prior reading the absorption of the color-reaction at 405 nm, or (ii) the fluorescence intensity is measured directly (absorption and emission spectra being dependent on the fluorochrome used for antibody conjugation). Applying linear regression analyses on the signals obtained from the used standard-calibration with recombinant Hla, the amount of Hla in the test-samples can be quantified.

### (Quantitative real-time RT-) PCR-based detection of the alpha-hemolysin gene and its expression

As a template for the (quantitative real-time RT-) PCR-based detection of *hla* (the gene and/or transcription product) DNA or RNA is isolated from *S. aureus* (directly applying a colony-PCR or by applying high-quality purification methods prior subjecting the template to PCR). Using gene-specific primers (to amplify the *hla* gene and a housekeeping gene such as 16S RNA, designed using molecular biology techniques as known in the art) in a PCR-based amplification reaction, the *hla* gene can be amplified and the amplicon can be visualized (e.g. by running the reaction on an agarose gel stained with an intercalating DNA dye). For the quantitative (q)-PCR the transcription-product of *hla* (an RNA) is transcribed into cDNA (reverse transcription, RT) and subsequently amplified. For quantification in the q-PCR step, fluorescence probes (e.g. SYBR green, TaqMan probes) are used, where the fluorescence-signal obtained during the exponential amplficiation correlates with the amount of DNA being amplified in real-time. This quantification allows the specific expression-profiling of the hla-gene in different biological samples but also in different growth phases of *S. aureus* and different growth conditions that might be tested.

### Functional assay for the detection of the alpha-hemolysin protein

A functional assay testing qualifying and semi-quantifying the expression of Hla is in line with the detailed description of the blood-agar hemolysis determination. In this assay, the Hla activity can be easily measured and a readout of 2+, 3+, or 4+ (Figure 1) is indicative for high Hla-activity and can be correlated to disease progression.

### Example 4: Diagnostic combination kit

A diagnostic combination kit may be used for detection of proteinaceous and non-proteinaceous bacterial factors associated with *S. aureus* infection and disease. This diagnostic kit can, for example, be used in a lateral flow immunochromatographic assays (such as dipstick tests) format which is exemplified as following.

Potential bacterial antigen targets to be tested for in a combined format could be (i) Alpha haemolysin as a biomarker for severity of a *S. aureus* infection and prediction for the onset of *S. aureus* disease in heavily colonized patients, (ii) PBP2a which is the methicillin-resistance marker allowing differentiation between MSSA and MRSA strains and (iii) cell-wall components such as lipoteichoic acid or wall-teichoic acid, or cell surface *S. aureus* specific Protein A which allow the confirmation of *S. aureus* colonization and/or specific detection of *S. aureus* also from patients co-infected with other Gram positive pathogens which might be detected in the sample taken. These antigens could for example be detected in ETA samples that are, for example routinely taken from ventilated patients in the intensive care unit by bronchoalveolar lavage. In an alternative setting, the antigens could also be detected from other bodily fluids that are routinely taken in hospital settings including blood, serum, sputum, urine and stool. Specific monoclonal antibodies would be used to detect the presence or absence of the antigens listed above either alone or in combination.

In the dipstick format, the test strip can consist of a ribbon made paper or plastic with pads which are impregnated with a substance or reagent such as a monospecific, monoclonal antibody, specifically reactive with the *S. aureus* antigens to be detected. A positive control could be included that enabled detection of an antigen specific for the bodily fluid being analysed to confirm correct functioning of the lateral flow assay. Dipsticks might be used as qualitative strips, which solely determine the presence or absence of *S. aureus* associated factors, or semi-quantitative strips which in addition to providing the qualitative readout, also provide an estimation of a quantitative result where a colour reaction is approximately proportional to the concentration of the substance being tested for in a sample. The strips are partly or completely immersed in the well mixed sample of the bodily fluid being analysed for a short time, extracted from the container, supporting the edge of the strip over the mouth of thereof to remove excess sample. This is followed by an incubation at room temperature for a few minutes prior reading the results by comparing the colours to a color scale provided together with the strip. For semi-quantiative readout the e.g. the intensity of the color reaction can be compared to a standardized colour-scale provided together with the strip and for example being reported as trace, 1+, 2+, 3+ and 4+ and/or an estimated amount of each detected factor in e.g. milligram per decilitre.

In a certain assay format (e.g., lateral flow format), strips are partially immersed in the well mixed sample of the bodily fluid being analyzed for a short time and extracted from the container. This is followed by an incubation at room temperature in order to enable sample flow to impregnated pads for a few minutes prior reading the results. Results are read by the operator and *S. aureus* markers are detected qualitatively as e.g. visible bands at the designated positions on the assay strip.

In certain assay formats, the readout can also be automated and thus be highly standardized in the clinic, for example using a digitalized system as a readout of the assay. As an example, the test sample is absorbed to the test-strip and transported by capillary-forces to the reaction field - here the reaction (mostly chemical) takes place and the electrons produced by this reaction are determined electrochemically (amperometry or colourimetry).

Alternatively the detecting antibodies are immobilized on a "sensor" and directly detecting the analyte.

### REFERENCES

1. Bassetti M, Taramasso L, Giacobbe DR, Pelosi P. Management of ventilator-associated pneumonia: epidemiology, diagnosis and antimicrobial therapy. Expert Rev Anti Infect Ther 2012; 10:585-596.
2. Valencia M, Torres A. Ventilator-associated pneumonia. Curr Opin Crit Care 2009; 15:30-35. Review.
3. Kollef MH, Hamilton CW, Ernst FR. Economic impact of ventilator-associated pneumonia in a large matched cohort. Infect Control Hosp Epidemiol 2012; 33:250-256.
4. Craven DE, Lei Y, Ruthazer R, Sarwar A, Hudcova J. Incidence and outcomes of ventilator-associated tracheobronchitis and pneumonia. Am J Med 2013; 126:542-549.
5. Dallas J, Skrupky L, Abebe N, Boyle WA 3rd, Kollef MH. Ventilator-associated tracheobronchitis in a mixed surgical and medical ICU population. Chest 2011; 139:513-518.
6. Grgurich PE, Hudcova J, Lei Y, Sarwar A, Craven DE. Diagnosis of ventilator-associated pneumonia: controversies and working toward a gold standard. Curr Opin Infect Dis 2013; 26:140-150.
7. Nseir S, Favory R, Jozefowicz E, Decamps F, Dewavrin F, Brunin G, Di Pompeo C, Mathieu D, Durocher A. Antimicrobial treatment for ventilator-associated tracheobronchitis: a randomized, controlled, multicenter study. Crit Care 2008; 12:R62.
8. Watkins RR, David MZ, Salata RA. Current concepts on the virulence mechanisms of meticillin-resistant Staphylococcus aureus. J Med Microbiol 2012; 61:1179-1193.
9. Tong SY, Chen LF, Fowler VG Jr. Colonization, pathogenicity, host susceptibility, and therapeutics for Staphylococcus aureus: what is the clinical relevance? Semin Immunopathol 2012; 34:185-200.
10. Berube BJ, Bubeck Wardenburg J. Staphylococcus aureus α-toxin: nearly a century of intrigue. Toxins (Basel) 2013; 5:1140-1166.
11. Bubeck Wardenburg J, Schneewind O. Vaccine protection against Staphylococcus aureus pneumonia. J Exp Med 2008; 205:287-294.
12. Foletti D, Strop P, Shaughnessy L, Hasa-Moreno A, Casas MG, Russel M, Bee C, Wu S, Pham A, Zeng Z, Pons J, Rajpal A, Shelton D. Mechanism of action and in vivo efficacy of a human-derived antibody against Staphylococcus aureus α-hemolysin. J Mol Biol 2013; 425:1641-1654.
13. Hua L, Hilliard JJ, Shi Y, Tkaczyk C, Cheng LI, Yu X, Datta V, Ren S, Feng H, Zinsou R, Keller A, O'Day T, Du Q, Cheng L, Damschroder M, Robbie G, Suzich J, Stover CK, Sellman BR. Assessment of anti-alpha toxin mAb for prevention and treatment of Staphylococcus aureus induced pneumonia. Antimicrob Agents Chemother 2013; 58:1108-1117
14. Cheung GYC, Otto M. The potential use of toxin antibodies as a strategy for controlling acute Staphylococcus aureus infections. Expert Opin Ther Targets 2012; 16:601-612.
15. McNamara PJ. Genetic Manipulation of Staphylococcus aureus. In: Lindsay JA, editor. Staphylococcus: Molecular Genetics, 1st ed., Norfolk, UK: Caister Academic Press; 2008. p. 100-102.
16. Kato K, Sugai M. A simple method of markerless gene deletion in Staphylococcus aureus. J. Microbiol. Methods 2011; 87:76-81.

## Claims

1. A method for the prediction of ventilator-associated pneumonia (VAP) in a ventilated subject heavily colonized by methicillin susceptible *Staphylococcus aureus* (MSSA) and asymptomatic, comprising the step of determining the alpha haemolysin level in a biological sample of said subject as compared to a predetermined alpha haemolysin level, wherein an elevated alpha haemolysin level is indicative of the risk of VAP disease onset, wherein the predetermined alpha haemolysin level is higher than a reference value of a *S. aureus* strain expressing no alpha haemolysin.

2. The method of claim 1, wherein the subject is colonized by MSSA as determined by a method of determining the presence of the bacterium or a bacterial marker indicative of MSSA, preferably by a method of determining the gene expressing said marker, an expression product of said gene, or using standard microbiological techniques.

3. The method of claim 1 or 2, wherein the subject is colonized in the nose or nasopharynx, and the subject is undergoing intubation.

4. The method of any of claims 1 to 3, wherein the subject is a mechanically ventilated patient and heavily colonized with *S. aureus* in the lower airways.

5. The method of any of claims 1 to 4, wherein the biological sample is a body fluid or tissue sample, preferably selected from the group consisting of a blood sample, stool sample, skin sample, urine sample, cerebrospinal fluid, and a respiratory tract specimen such as endotracheal aspirates, pleural fluid, lung tap, nasal swab or sputum.

6. The method of any of claims 1 to 5, wherein said determination of the alpha haemolysin level comprises a semi-quantitative or quantitative measurement of alpha haemolysin expression or activity.

7. The method of any of claims 1 to 6, wherein the level of alpha haemolysin is determined by an immunoassay, preferably any of ELISA, CIA, RIA, IRMA, agglutination assay, immunochromatography, preferably lateral flow immunochromatographic assays, such as dipstick tests, and Western-blot, mass-spectrometry, NMR, or a method of determining corresponding DNA or RNA indicative of alpha haemolysin, preferably employing a nucleic acid hybridization assay or a nucleic acid amplification assay.

8. The method of any of claims 1 to 7, wherein said predetermined alpha haemolysin level is obtained from a *S. aureus* strain expressing low, medium or high alpha haemolysin.

9. The method of any of claims 1 to 8, which further comprises the measurement of at least one additional marker indicative of *S. aureus* colonization.

10. The method of claim 9, wherein said measurement of the at least one additional marker comprises the determination of a gene expressing any *S. aureus* specific bacterial component or the respective gene expression product of any *S. aureus* specific bacterial component, other than alpha haemolysin, preferably selected from the group consisting of
i) *S. aureus* Protein A;
ii) a *S. aureus* antibiotic resistance marker preferably at least any of PBP2a, the *mecA* gene or an expression product of the *mecA* gene;
iii) any other *S. aureus* antigen including a *S. aureus* cytotoxin, preferably PVL/LukSF, LukGH, gamma-haemolysin, LukED; *S. aureus* virulence factors, secreted and surface proteins; or any *S. aureus* specific compounds such as lipoteichoic acid, and
iv) a combination of any of the bacterial components of i), ii) and/or iii), preferably determining as additional markers at least *S. aureus* Protein A and PBP2a.

11. Use of alpha haemolysin as an *in vitro* marker for determining the risk of VAP disease in a ventilated asymptomatic subject heavily colonized by MSSA.

12. *In vitro* use of a diagnostic composition comprising a specific detection molecule for alpha haemolysin for determining the risk of VAP disease in a ventilated subject heavily colonized with MSSA, wherein the detection molecule is an alpha haemolysin binder selected from the group consisting of an antibody, antibody fragment, cellular receptor or ligand, or a nucleotide sequence hybridizing to the alpha haemolysin gene or an expression product of said gene.

13. Use of an alpha haemolysin diagnostic kit in a method of any of claims 1 to 10, wherein the diagnostic kit comprises one or more reagents capable of specifically reacting with an alpha haemolysin expression product and/or a specific detection molecule suitable for determining the level of alpha haemolysin, which is provided in the storage-stable form, preferably as a packaged unit.

14. The use according to claim 13, wherein the diagnostic kit is a diagnostic combination kit comprising
a) a kit comprising one or more reagents capable of specifically reacting with an alpha haemolysin expression product and/or a specific detection molecule suitable for determining the level of alpha haemolysin; and
b) a kit comprising a reagent and/or a specific detection molecule suitable for determining a gene expressing a *S. aureus* specific bacterial component or the respective gene expression product of a *S. aureus* specific bacterial component, other than alpha haemolysin, preferably selected from the group consisting of
i) *S. aureus* Protein A;
ii) a *S. aureus* antibiotic resistance marker preferably at least any of PBP2a, the *mecA* gene or an expression product of the *mecA* gene;
iii) any other *S. aureus* antigen including a *S. aureus* cytotoxin, preferably PVL/LukSF, LukGH, gamma-haemolysin, LukED; *S. aureus* virulence factors, secreted and surface proteins; or any *S. aureus* specific compounds such as lipoteichoic acid, and
iv) a combination of any of the bacterial components of i), ii) and/or iii), preferably determining as additional markers at least *S. aureus* Protein A and PBP2a.

## Patentansprüche

1. Verfahren zur Vorhersage einer beatmungsassoziierten Pneumonie (ventilator-associated pneumonia, VAP) in einem beatmeten Subjekt, das stark mit methicillin-sensitivem *Staphylococcus aureus* (MSSA) besiedelt und symptomlos ist, umfassend den Schritt des Bestimmens des Alpha-Hämolysin-Niveaus in einer biologischen Probe des Subjekts verglichen mit einem vorbestimmten Alpha-Hämolysin-Niveau, wobei ein erhöhtes Alpha-Hämolysin-Niveau auf das Risiko des Einsetzens einer VAP-Erkrankung hinweist, wobei das vorbestimmte Alpha-Hämolysin-Niveau höher als ein Referenzwert eines *S*. *aureus-Stammes* ist, der kein Alpha-Hämolysin exprimiert.

2. Verfahren nach Anspruch 1, wobei das Subjekt mit MSSA besiedelt ist, bestimmt durch ein Verfahren zur Bestimmung des Vorliegens des Bakteriums oder eines bakteriellen Markers, der auf MSSA hinweist, vorzugsweise durch ein Verfahren zur Bestimmung des Gens, das den Marker exprimiert, eines Expressionsprodukts des Gens, oder unter Verwendung von mikrobiologischen Standardtechniken.

3. Verfahren nach Anspruch 1 oder 2, wobei das Subjekt in der Nase oder dem Nasopharynx besiedelt ist, und das Subjekt einer Intubation unterzogen wird.

4. Verfahren nach einem der Ansprüche 1 bis 3, wobei das Subjekt ein mechanisch beatmeter Patient ist und in den unteren Luftwegen stark mit *S. aureus* besiedelt ist.

5. Verfahren nach einem der Ansprüche 1 bis 4, wobei die biologische Probe eine Körperflüssigkeit oder eine Gewebeprobe ist, vorzugsweise ausgewählt aus der Gruppe bestehend aus einer Blutprobe, Stuhlprobe, Hautprobe, Urinprobe, Zerebrospinalfluid und einer Probe aus den Atemwegen, wie Endotrachealaspiraten, Pleuraflüssigkeit, Lungenpunktion, Nasenabstrich oder Sputum.

6. Verfahren nach einem der Ansprüche 1 bis 5, wobei die Bestimmung des Alpha-Hämolysin-Niveaus eine semiquantitative oder quantitative Messung der Alpha-Hämolysin-Expression oder -Aktivität umfasst.

7. Verfahren nach einem der Ansprüche 1 bis 6, wobei das Niveau von Alpha-Hämolysin durch ein Immunoassay bestimmt wird, vorzugsweise ein beliebiges aus ELISA, CIA, RIA, IRMA, Agglutinierungsassay, Immunochromatographie, vorzugsweise immunochromatographischen Querstrom-Assays, wie Eintauchstreifentests, und Western-Blot, Massenspektrometrie, NMR oder ein Verfahren zum Bestimmen der entsprechenden DNA oder RNA, die auf Alpha-Hämolysin hinweisen, vorzugsweise unter Einsatz eines Nukleinsäure-Hybridisierungstests oder eines Nukleinsäure-Amplifikationstests.

8. Verfahren nach einem der Ansprüche 1 bis 7, wobei das vorbestimmte Alpha-Hämolysin-Niveau von einem S. aureus-Stamm erhalten wird, der ein geringes, mittleres oder hohes Niveau an Alpha-Hämolysin exprimiert.

9. Verfahren nach einem der Ansprüche 1 bis 8, welches ferner die Messung mindestens eines zusätzlichen Markers umfasst, der auf eine Besiedelung mit *S. aureus* hinweist.

10. Verfahren nach Anspruch 9, wobei die Messung des mindestens einen zusätzlichen Markers die Bestimmung eines Gens, das eine beliebige für *S. aureus* spezifische bakterielle Komponente exprimiert, oder des jeweiligen Genexpressionsprodukts einer beliebigen für *S. aureus* spezifischen bakteriellen Komponente, die nicht Alpha-Hämolysin ist, umfasst, vorzugsweise ausgewählt aus der Gruppe bestehend aus
i) *S. aureus*-Protein A;
ii) einem Antibiotika-Resistenzmarker von *S. aureus,* vorzugsweise mindestens einem von PBP2a, dem mecA-Gen oder einem Expressionsprodukt des *mecA-*Gens;
iii) einem beliebigen anderen *S. aureus*-Antigen, darunter ein *S*. *aureus-*Cytotoxin, vorzugsweise PVL/LukSF, LukGH, Gamma-Hämolysin, LukED; Virulenzfaktoren, sekretierten und Oberflächen-Proteinen von *S*. *aureus*; oder beliebigen für *S. aureus* spezifischen Verbindungen wie Lipoteichonsäure, und
iv) einer Kombination beliebiger der bakteriellen Komponenten aus i), ii) und/oder iii), wobei als zusätzliche Marker vorzugsweise mindestens *S. aureus*-Protein A und PBP2a bestimmt werden.

11. Verwendung von Alpha-Hämolysin als einen *in vitro*-Marker zur Bestimmung des Risikos einer VAP-Erkrankung in einem beatmeten, symptomlosen Subjekt, das stark mit MSSA besiedelt ist.

12. *In vitro*-Verwendung einer diagnostischen Zusammensetzung, umfassend ein spezifisches Nachweismolekül für Alpha-Hämolysin, zur Bestimmung des Risikos einer VAP-Erkrankung in einem beatmeten Subjekt, das stark mit MSSA besiedelt ist, wobei das Nachweismolekül ein Alpha-Hämolysin-Binder, ausgewählt aus der Gruppe bestehend aus einem Antikörper, Antikörperfragment, zellulären Rezeptor oder Ligand, ist, oder eine Nukleotidsequenz, die mit dem Alpha-Hämolysin-Gen oder einem Expressionsprodukt des Gens hybridisiert.

13. Verwendung eines Alpha-Hämolysin-Diagnosekit in einem Verfahren nach einem der Ansprüche 1 bis 10, wobei das Diagnosekit ein oder mehrere Reagenzien umfasst, die in der Lage sind, spezifisch mit einem Alpha-Hämolysin-Expressionsprodukt zu reagieren, und/oder ein spezifisches Nachweismolekül, das zur Bestimmung des Niveaus von Alpha-Hämolysin geeignet ist, und in der lagerstabilen Form, vorzugsweise als eine verpackte Einheit, vorgesehen ist.

14. Verwendung nach Anspruch 13, wobei das Diagnosekit ein Kombinations-Diagnosekit ist, umfassend
a) ein Kit, umfassend ein oder mehrere Reagenzien, die in der Lage sind, spezifisch mit einem Alpha-Hämolysin-Expressionsprodukt zu reagieren, und/oder ein spezifisches Nachweismolekül, das zur Bestimmung des Niveaus von Alpha-Hämolysin geeignet ist; und
b) ein Kit, umfassend ein Reagens und/oder ein spezifisches Nachweismolekül, das zur Bestimmung eines Gens, das eine für *S. aureus* spezifische bakterielle Komponente exprimiert, oder des jeweiligen Genexpressionsprodukts einer für *S. aureus* spezifischen bakteriellen Komponente, die nicht Alpha-Hämolysin ist, geeignet ist, vorzugsweise ausgewählt aus der Gruppe bestehend aus
i) *S. aureus-*Protein A;
ii) einem Antibiotika-Resistenzmarker von *S. aureus,* vorzugsweise mindestens einem von PBP2a, dem *mecA*-Gen oder einem Expressionsprodukt des *mecA*-Gens;
iii) einem beliebigen anderen *S*. *aureus*-Antigen, darunter ein *S*. *aureus-*Cytotoxin, vorzugsweise PVL/LukSF, LukGH, Gamma-Hämolysin, LukED; Virulenzfaktoren, sekretierten und Oberflächen-Proteinen von *S*. *aureus*; oder beliebigen für *S. aureus* spezifischen Verbindungen wie Lipoteichonsäure, und
iv) einer Kombination beliebiger der bakteriellen Komponenten aus i), ii) und/oder iii), wobei als zusätzliche Marker vorzugsweise mindestens *S. aureus-*Protein A und PBP2a bestimmt werden.

## Revendications

1. Procédé de prévision d'une pneumonie associée au ventilateur (PAV) chez un sujet ventilé fortement colonisé par *Staphylococcus aureus* sensible à la méthicilline (SASM) et asymptomatique, comprenant l'étape de détermination du taux d'alpha hémolysine dans un échantillon biologique dudit sujet comparativement à un taux d'alpha hémolysine prédéfini, un taux d'alpha hémolysine élevé indiquant le risque d'apparition de maladie PAV, ledit taux d'alpha hémolysine prédéfini étant supérieur à une valeur de référence d'une souche de *S. aureus* n'exprimant pas d'alpha hémolysine.

2. Procédé selon la revendication 1, dans lequel le sujet est colonisé par SASM comme cela est déterminé par un procédé de détermination de la présence de la bactérie ou d'un marqueur bactérien indicateur de SASM, de préférence par un procédé de détermination du gène exprimant ledit marqueur, un produit d'expression dudit gène, ou à l'aide de techniques microbiologiques standards.

3. Procédé selon la revendication 1 ou 2, dans lequel le sujet est colonisé dans le nez ou le nasopharynx, et le sujet est en cours d'intubation.

4. Procédé selon l'une quelconque des revendications 1 à 3, dans lequel le sujet est un patient ventilé mécaniquement et fortement colonisé par *S. aureus* dans les voies respiratoires inférieures.

5. Procédé selon l'une quelconque des revendications 1 à 4, dans lequel l'échantillon biologique est un échantillon de fluide corporel ou de tissu, de préférence choisi dans le groupe constitué par un échantillon de sang, un échantillon de selles, un échantillon de peau, un échantillon d'urine, le liquide céphalorachidien et un échantillon du système respiratoire tel que des aspirats endotrachéaux, du liquide pleural, une biopsie de poumon, un écouvillonnage nasal ou un crachat.

6. Procédé selon l'une quelconque des revendications 1 à 5, dans lequel ladite détermination du taux d'alpha hémolysine comprend une mesure quantitative ou semi-quantitative de l'expression ou de l'activité de l'alpha hémolysine.

7. Procédé selon l'une quelconque des revendications 1 à 6, dans lequel le taux d'alpha hémolysine est déterminé par un dosage immunologique, de préférence l'un quelconque d'un dosage ELISA, CIA, RIA, IRMA, un dosage d'agglutination, une immunochromatographie, de préférence des dosages immunochromatographiques à écoulement latéral, tels que des tests par bandelettes réactives, et le transfert Western, la spectrométrie de masse, la RMN, ou un procédé de détermination d'ADN ou d'ARN correspondant indicateur de l'alpha hémolysine, de préférence employant un dosage par hybridation d'acide nucléique ou un dosage par amplification d'acide nucléique.

8. Procédé selon l'une quelconque des revendications 1 à 7, dans lequel ledit taux d'alpha hémolysine prédéfini est obtenu à partir d'une souche de *S. aureus* exprimant faiblement, moyennement ou fortement l'alpha hémolysine.

9. Procédé selon l'une quelconque des revendications 1 à 8, qui comprend en outre la mesure d'au moins un marqueur supplémentaire indicateur d'une colonisation par *S. aureus.*

10. Procédé selon la revendication 9, dans lequel ladite mesure dudit au moins un marqueur supplémentaire comprend la détermination d'un gène exprimant un quelconque composant bactérien spécifique à *S. aureus* ou le produit d'expression génétique respectif d'un quelconque composant bactérien spécifique à *S. aureus*, différent de l'alpha hémolysine, de préférence choisi dans le groupe constitué par
i) la protéine A de *S*. *aureus* ;
ii) un marqueur de résistance aux antibiotiques de *S. aureus,* de préférence au moins l'un quelconque de PBP2a, du gène *mecA* ou d'un produit d'expression du gène *mecA* ;
iii) tout autre antigène de *S*. *aureus* dont une cytotoxine de *S. aureus,* de préférence PVL/LukSF, LukGH, la gamma-hémolysine, LukED ; des facteurs de virulence de *S. aureus,* des protéines sécrétées et de surface ; ou tous les composés spécifiques à *S. aureus* tels que l'acide lipotéichoïque, et
iv) une combinaison de l'un quelconque des composants bactériens de i), ii) et/ou iii), de préférence déterminant comme marqueurs supplémentaires au moins la protéine A de *S. aureus* et PBP2a.

11. Utilisation de l'alpha hémolysine comme marqueur *in vitro* pour déterminer le risque de maladie PAV chez un sujet asymptomatique ventilé fortement colonisé par SASM.

12. Utilisation *in vitro* d'une composition de diagnostic comprenant une molécule de détection spécifique pour l'alpha hémolysine pour déterminer le risque de maladie PAV chez un sujet ventilé fortement colonisé par SASM, ladite molécule de détection étant un liant d'alpha hémolysine choisi dans le groupe constitué par un anticorps, un fragment d'anticorps, une récepteur ou un ligand cellulaire, ou une séquence nucléotidique s'hybridant au gène d'alpha hémolysine ou un produit d'expression dudit gène.

13. Utilisation d'un kit de diagnostic d'alpha hémolysine dans un procédé selon l'une quelconque des revendications 1 à 10, ledit kit de diagnostic comprenant un ou plusieurs réactifs capables de réagir spécifiquement avec un produit d'expression d'alpha hémolysine et/ou une molécule de détection spécifique appropriée pour la détermination du taux d'alpha hémolysine, qui est fourni sous la forme stable au stockage, de préférence sous forme d'unité emballée.

14. Utilisation selon la revendication 13, ledit kit de diagnostic étant un kit de combinaison de diagnostic comprenant
a) un kit comprenant un ou plusieurs réactifs capables de réagir spécifiquement avec un produit d'expression d'alpha hémolysine et/ou une molécule de détection spécifique appropriée pour la détermination du taux d'alpha hémolysine, et
b) un kit comprenant un réactif et/ou une molécule de détection spécifique appropriée pour la détermination d'un gène exprimant un composant bactérien spécifique à *S. aureus* ou le produit d'expression génétique respectif d'un composant bactérien spécifique à *S. aureus,* différent de l'alpha hémolysine, de préférence choisi dans le groupe constitué par
i) la protéine A de *S. aureus* ;
ii) un marqueur de résistance aux antibiotiques de *S. aureus,* de préférence au moins l'un quelconque de PBP2a, du gène *mecA* ou d'un produit d'expression du gène *mecA* ;
iii) tout autre antigène de *S. aureus* dont une cytotoxine de *S. aureus,* de préférence PVL/LukSF, LukGH, la gamma-hémolysine, LukED ; des facteurs de virulence de *S. aureus,* des protéines sécrétées et de surface ; ou tous les composés spécifiques à *S. aureus* tels que l'acide lipotéichoïque, et
iv) une combinaison de l'un quelconque des composants bactériens de i), ii) et/ou iii), de préférence déterminant comme marqueurs supplémentaires au moins la protéine A de *S. aureus* et PBP2a.
